# EUROPEAN PATENT APPLICATION

(11) **EP 3 617 964 A1**
(43) Date of publication of application: **04.03.2020**
(21) Application number: 19185442.1
(22) Date of filing: 10.07.2019
(51) Int. Cl.: G06Q 10/06

(54) **PERFORMANCE MEASUREMENT DEVICE, PERFORMANCE MEASUREMENT METHOD AND PERFORMANCE MEASUREMENT PROGRAM**

(30) Priority: 31.08.2018 JP 2018163162
(71) Applicant: OMRON Corporation, Shiokoji-dori, Shimogyo-ku Kyoto-shi Kyoto 600-8530 (JP)
(72) Inventor: Akatsuka, Mayumi, Kyoto, 619-0283 (JP); Kotake, Yasuyo, Kyoto, 619-0283 (JP)
(74) Representative: Hoffmann Eitle

(57) **Abstract**

A performance measurement device according to an aspect of the invention is configured to acquire sensing data obtained by measuring an activity relating to at least one of sensory activity and physical activity for a relevant worker; analyze the sensing data acquired and thereby calculate a performance index for each of the basic operations; compare the performance index required for each of the basic operations in a work step and the performance index calculated for each of the basic operations; and assign the worker a second work step where the performance index calculated for each of the basic operations satisfies a predetermined criterion in relation to the required performance index for each of the basic operations.

## Description

### CROSS-REFERENCE TO RELATED APPLICATIONS

This application claims the benefit of Japanese Patent Application No. 2018-163162 filed June 14, 2018.

### FIELD

The present invention relates to a performance measurement device, a performance measurement method and a performance measurement program.

### BACKGROUND

Japanese Patent Publication No. 2007-293690 proposes a personnel assignment system for assigning workers to work processes. More specifically, the personnel assignment system proposed in JP 2007-293690 A calculates a takt time on the basis of the number of production units scheduled, assigns basic operations to a plurality of work steps on the basis of the takt time and the standard work time for each basic operation; and based on the takt time and the cycle time of each basic step for each of the workers, determines how to assign the workers to each of the work steps assigned basic operations. The personnel assignment system thus assigns workers to work steps so that there are no impediments to the overall production plan.

### Technical Problem

The inventors discovered that existing systems, such as the one described in JP 2007-293690 A have the following drawbacks. That is, in the conventional system the processing time for a given work step is used as a measure of ability (level of proficiency) of a person performing work steps. In other words, existing systems evaluate shorter processing times as indicating a better ability at performing a work step, while evaluating longer processing times as indicating a worse ability at performing the work step. The processing time for a work step may be used for objective and quantitative assessment of whether or not a person can complete said work step within a predetermined time.

However, the processing time for a work step does not include information on the person's actual process of performing the work step. That is, the processing time for a work step does not include information that objectively and quantitatively indicates what ability a person has in performing each work step (in other words, the kind of skills the person possesses in relation to each work step). Therefore, it has not been possible to objectively assess from the information indicating the processing time for a work step, what a person is careful about or what actions must be performed in order to suitably execute the work step.

Thus, the inventors discovered that existing systems are unable to provide objective and repeatable techniques for evaluating a person's abilities in relation to a work step. Additionally, it tends to be difficult to evaluate the abilities of a target worker in each work step if the processing time is not measured for all the work steps performed by the target worker. That is, it is unclear whether a worker capable of completing a target work step in a predetermined time is capable of completing another work step within the same predetermined time without measuring the worker's processing time in relation to said other work step. Thus, the inventors have discovered the techniques currently available cannot appropriately determine and assign a work step to a worker in order to systematically improve and maintain the productivity and the efficiency of a production line.

Given the forgoing, an aspect of the present invention aims to address these problems by providing techniques for objectively and quantitatively measuring the ability of a person to perform a work step in order to appropriately determine and assign a work step to a worker.

### SUMMARY

To address the above described disadvantages, an embodiment of the present invention is configured as follows.

A performance measurement device according to an aspect of the present invention includes a data acquisition unit configured to acquire sensing data obtained from one or a plurality of sensors configured to measure an activity relating to at least one of sensory activity and physical activity of a worker while the worker executes a first work step among a plurality of work steps performed at a production site, each work step including a plurality of basic operations; an index calculating unit configured to analyze the sensing data acquired and thereby use the sensing data to calculate a performance index for the worker in each of the basic operations, the performance index indicating a degree of performance of the basic operations achieved through said activity; a comparator unit configured to compare the performance index required for each of the basic operations in a work step to suitably accomplish the work step, and the performance index calculated for the worker in each of the basic operations; a work assignment unit configured to assign the worker a second work step from the plurality of work steps on the basis of the results of the comparison, where the performance index calculated for the worker in each of the basic operations satisfies a predetermined criterion in relation to the performance index required for each of the basic operations; and an output unit configured to output the result of the assignment.

A performance measurement device configured as above described uses one or a plurality of sensors to measure at least one of sensory activity and physical activity of a worker while the worker is executing a first work step. Sensory activity involves recognizing the attributes of an object such as the position, shape, size, and texture; and allows for some kind of assessment to be made based on the result of the recognition. Sensory activity is achieved through using primarily sensory systems for vision, hearing, touch, or the like. Physical activity involves moving the body to change the positional relationship with an object, and involves physically affecting an object. Physical activity is achieved through using the musculoskeletal system which is made up primarily the tissues and organs related to moving the body, the such as the bones, muscles, joints, and nerves. That is, the sensory activity and physical activity each represent the behavior of the sensory system and the musculoskeletal system as physiological parameters. Therefore, the sensory activity and the physical activity can be measured by way of measuring the behavior of the sensory system and the musculoskeletal system using sensors.

The sensing data obtained through the above measurement can indicate the performance of at least one of the sensory activity and the physical activity. The performance of the sensory activity and the physical activity correlates to the results of whether or not a work step can be suitably accomplished. That is, the higher the performance of the sensory activity and the physical activity, the greater the ability to accomplish a work step, and the lower the performance of the sensory activity and the physical activity, the lesser the ability to accomplish a work step. Therefore, a performance measurement device according to the above configuration analyzes the sensing data obtained to calculate a performance index from the sensing data for each of the basic operations included in a first work step.

The performance index can be used to objectively and quantitatively indicate the ability of a worker with respect to basic operations. Accordingly, performance indexes obtained for the basic operations included in a first work step can objectively and quantitatively indicate ability, which is how a worker performs that first work step. For example, it is possible to indicate in which of a plurality of basic operations contained in a first work step that a worker does well or does poorly.

The performance measurement device configured as above described compares the performance index required for each of the basic operations in a work step for suitably accomplishing the work step, and the performance index calculated for a worker in each of the basic operations. The performance measurement device configured as above described assigns the worker a second work step on the basis of the comparison results where the performance index calculated for the worker in each of the basic operations satisfies a predetermined criterion in relation to the required performance index for the basic operations. Thus, the performance measurement device configured as above described uses the results of evaluating the performance index to assign the aforementioned worker a work step for which the worker is suited.

Accordingly, a performance measurement device, configured as above described, is capable of objectively and quantitatively measuring the ability of a person to perform a work step, and to appropriately assign a work step to a worker. Therefore, it is possible to systematically improve or maintain the productivity and efficiency of the production line at the production site.

The work steps performed at a production site are achieved through a series of sensory activities and physical activities. Thus, the process of executing a work step may be expressed as a combination of a plurality of types of sensory activity and physical activity. The basic operations may then be defined from a combination of sensory activity and physical activity. Work steps are preferably repeatable. The combination of the same types of sensory activity and physical activity may manifest each time a work step is executed when a person repeatedly executes the same work step. A segment expressing a combination of the same types of activity within executing these work steps may be output as a segment where a common basic operation (i.e. the same type of basic operation) is being performed. Each of the basic operations included in a work step may be ordered in a time series. When a person is somewhat used to a particular work step, the result of performing the basic operation may be entered into the subsequent basic operation. The degree of performance in the basic operations, which can be represented by a performance index, corresponds to the level of ability in suitably accomplishing each of the basic operations. A work step is not particularly limited, and may be selected is appropriate in accordance with the production site at which an embodiment of the invention is being adopted. A work step at a production site may be soldering, fitting cases, inserting parts for insertion, inspection, packaging, or the like. A second work step that is assigned to a worker may be different from or the same as the first work step executed by the worker while the performance index was being calculated.

The type of sensor is not particularly limited, as long as the sensor is capable of measuring a physiological parameter relating to at least one of the sensory activity and the physical activity of the worker; the type of sensor may be selected as appropriate in accordance with the form of implementation. The behavior of the sensory systems may be expressed for instance through brain waves, cerebral blood flow, pupil diameter, gaze direction, facial expression, voice, electrocardiogram, blood pressure, electromyograph, a galvanic skin reflex (GSR), or the like. Therefore, the one or plurality of sensors for measuring sensory activity may be for example: an electroencephalographic meter (EEG); a magnetoencephalographic meter (MEG); a magnetic resonance imaging device configured to use functional magnetic resonance imaging (fMRI) to take images of the blood flow in relation to brain activity; a brain activity measurement device configured to use functional near infrared spectroscopy (fNIRS) to measure cerebral blood flow; a gaze sensor configured to measure pupil diameter and gaze direction; an electrooculographic sensor; a microphone; an electrocardiographic meter, a blood pressure meter; an electromyographic sensor; a galvanic skin reflex meter; and a camera. In contrast, the behaviors and physical activities may be expressed with the musculoskeletal system, such as the finger, hand, leg, neck, waist, joints, or muscles. Therefore, the one or plurality of sensors for measuring physical activity may be for instance a camera, motion capture device, load cell or a combination of these.

According to the above aspect, the data acquisition unit may repeatedly acquire sensing data for the worker; and the index calculating unit may calculate the performance index for the worker in each of the basic operations from the sensing data each time sensing data is acquired. The performance measurement device of the above mentioned aspect may further include a history creation unit configured to accumulate the performance indexes calculated for a worker in each of the basic operations to thereby create a history of performance indexes for the worker in each of the basic operations; The index calculating unit may predict a future performance index for the worker in each of the basic operations from past performance indexes represented by the history created; and the comparator unit may compare the performance index required for each of the basic operations in each of the work steps and the future performance index predicted for the worker in each of the basic operations. The aforementioned configuration predicts a future performance index for a worker and assigns the worker a work step that is compatible with the results of the prediction. Thus, the aforementioned configuration can more appropriately assign workers to work steps.

In a performance measurement device according to the above aspect, the data acquisition unit may use one or a plurality of sensors to measure the activity of a skilled operator able to suitably accomplish a third work step among the plurality of work steps while the skilled operator executes the third work step to thereby acquire sensing data; and the index calculating unit may analyze the sensing data acquired from the skilled operator to thereby calculate a performance index for the skilled operator in each of the basic operations. The performance measurement device according to the above aspect may further include a registration unit configured to register the performance index calculated for the skilled operator in each of the basic operations in the third work step as the performance index required for each of the basic operations. Given this configuration, it is possible to suitably establish a performance index required for each of the basic operations to suitably accomplish a work step; thus, it is possible to precisely determine whether or not a worker can suitably accomplish a target work step. The third work step may be identical to any of the first work step and the second work step, or may be different from the first work step and the second work step.

The performance measurement device according to the above aspect is configured so that each of the basic operations is defined to include a human cognitive process for at least one cycle; and sensing data is obtained by using a plurality of sensors to measure the sensory activity and the physical activity of the worker. Given this configuration, it is possible to appropriately define each of the basic operations included in a work step, and to appropriately acquire sensing data used for calculating a performance index for each of the basic operations. It is thereby possible to precisely measure the ability of a person to perform a work step.

The performance measurement device according to the above aspect is configured so that analyzing the sensing data includes evaluating at least any one of the correctness, speed, stability, and rhythm of executing the basic operations; and the index calculating unit calculates the performance index for the worker in each of the basic operations in accordance with the result of the evaluation. Given this configuration, it is possible to appropriately calculate a performance index for each of the basic operations whereby it is possible to precisely measure the ability of a person to accomplish a work step.

The performance measurement device according to the above aspect is configured so that analyzing the sensing data includes: converting the sensing data into time series feature data; analyzing the time series feature data to thereby identify the execution time, time overlap, number of executions, and execution order of the basic operations; and evaluating the correctness, speed, stability, and rhythm of execution for each of the basic operations on the basis of the execution time, time overlap, number of executions, and execution order for the basic operations; and the index calculating unit calculates the performance index for each of the basic operations in accordance with the result of the evaluation. Given this configuration, it is possible to appropriately calculate a performance index for each of the basic operations whereby it is possible to precisely measure the ability of a person to accomplish a work step.

The performance measurement device according to the above aspect may also be configured so that the work assignment unit determines whether or not the difference between the performance index calculated for a worker in each of the basic operations is at or below a predetermined value compared to the performance index required for each of the basic operations in any work step among the plurality of work steps; and assigns the worker any work step as a second work step on determining that the difference between a performance index required for each of the basic operations in said work step is at or below the performance index calculated for the worker in each of the basic operations. Given this configuration it is possible to determine and assign a work step to a worker to thus systematically improve and maintain the productivity of a production line.

The performance measurement device according to the above aspect may be configured so that the work assignment unit assigns the worker a work step from among the plurality of work steps as said second work step where the performance index calculated for the worker in at least any basic operation among the plurality of basic operations is lower than the performance index required for said basic operation, and the difference between the performance index calculated for the worker in each of the basic operations and the performance index required for each of the basic operations is a minimum. Given this configuration it is possible to determine and assign a work step to a worker to thus systematically improve and maintain the productivity of a production line while encouraging mastery of a target basic operation.

The performance measurement device according to the above aspect is configured so that the one or plurality of sensors is made up one or a combination of a camera, a microphone, an electroencephalography meter, a magnetoencephalographic meter, a magnetic resonance imaging device, an electrocardiography meter, a blood pressure meter, a galvanic skin reflex meter, an electromyographic sensor, a load cell, a motion capture device, a brain activity measurement device, a gaze sensor, and an electrooculographic sensor. Given this configuration, it is possible to appropriately acquire the sensing data used for calculating a performance index for each of the basic operations.

As another implementation of the performance measurement device of the above embodiments, one aspect of the present invention may be an information processing method or a program that implements the above configuration, or a computer readable recording medium that stores such a program. The computer readable recording medium may be a medium whereon the information for the program or the like is accumulated via electrical, magnetic, optical, mechanical, or chemical processes so that a computer, another device, or a machine is capable of reading the information recorded in the program or the like.

A performance measurement method according to an aspect of the present invention may cause a computer to execute steps including: acquiring sensing data obtained from one or a plurality of sensors configured to measure an activity relating to at least one of sensory activity and physical activity of a worker while the worker executes a first work step among a plurality of work steps performed at a production site, each work step including a plurality of basic operations; analyzing the sensing data acquired and thereby using the sensing data to calculate a performance index for the worker in each of the basic operations, the performance index indicating a degree of performance of the basic operations achieved through said activity; comparing the performance index required for each of the basic operations in a work step to suitably accomplish the work step, and the performance index calculated for the worker in each of the basic operations; assigning the worker a second work step from the plurality of work steps on the basis of the results of the comparison, where the performance index calculated for the worker in each of the basic operations satisfies a predetermined criterion in relation to the performance index required for each of said basic operations; and output of the result of the assignment.

A performance measurement program according to an aspect of the present invention may cause a computer to execute steps including: acquiring sensing data obtained from one or a plurality of sensors configured to measure an activity relating to at least one of sensory activity and physical activity of a worker while the worker executes a first work step among a plurality of work steps performed at a production site, each work step including a plurality of basic operations; analyzing the sensing data acquired and thereby using the sensing data to calculate a performance index for the worker in each of the basic operations, the performance index indicating a degree of performance of the basic operations achieved through said activity; comparing the performance index required for each of the basic operations in a work step to suitably accomplish the work step, and the performance index calculated for the worker in each of the basic operations; assigning the worker a second work step from the plurality of work steps on the basis of the results of the comparison, where the performance index calculated for the worker in each of the basic operations satisfies a predetermined criterion in relation to the performance index required for each of said basic operations; and output of the result of the assignment.

### Effects

Accordingly, embodiments of the present invention are capable of objectively and quantitatively measuring the ability of a person to perform a work step, and to appropriately assign a work step to a worker.

### BRIEF DESCRIPTION OF THE DRAWINGS

FIG. 1 schematically illustrates an example of a setting where the present invention may be adopted;
FIG. 2 schematically illustrates steps in the human cognitive process;
FIG. 3 schematically illustrates an example of a plurality of basic operations making up a work step;
FIG. 4 schematically illustrates an example of a hardware configuration for a performance measurement device according to an embodiment;
FIG. 5 schematically illustrates an example of a software configuration for a performance measurement device according to the embodiment;
FIG. 6 schematically illustrates an example of processing sequence during the measurement mode of a performance measurement device according to the embodiment;
FIG. 7 schematically illustrates an example of a processing sequence that analyzes sensing data according to the embodiment;
FIG. 8 schematically illustrates an example of sensing data;
FIG. 9 schematically illustrates an example of time series feature data;
FIG. 10 schematically illustrates an example of a process of mastering a work step;
FIG. 11A schematically illustrates an example of a method for evaluating the correctness of an execution of a basic operation;
FIG. 11B schematically illustrates an example of a method for evaluating the stability of an execution of a basic operation;
FIG. 11C schematically illustrates an example of a method for evaluating the speed of an execution of a basic operation;
FIG. 11D schematically illustrates an example of a method for evaluating the rhythm in an execution of a basic operation;
FIG. 12A illustrates an example of the relationship between the difficulty of a sensory activity and a standard value for a performance index;
FIG. 12B illustrates an example of the relationship between the difficulty of a physical activity and a standard value for a performance index;
FIG. 13 schematically illustrates an example of a proficiency database according to the embodiment;
FIG. 14 schematically illustrates an example of a processing sequence during a registration mode of a performance measurement device according to the embodiment;
FIG. 15 schematically illustrates an example of a processing sequence during an assignment mode of a performance measurement device according to the embodiment; and
FIG. 16 schematically illustrates an example of a work database according to the embodiment.

### DETAILED DESCRIPTION

An embodiment (below, "the embodiment") according to an aspect of the invention is described below on the basis of the drawings. However, at all points the embodiment described below is merely an example of the invention. It goes without saying that various modifications and variations are possible without departing from the scope of the invention. That is, specific configurations may be adopted as appropriate in accordance with the embodiment when implementing the invention. Note that the data that appears in the embodiment is described in natural language; however, these descriptions point to virtual languages, commands, parameters, machine language and the like that may be recognized by a computer.

### 1. Example Application

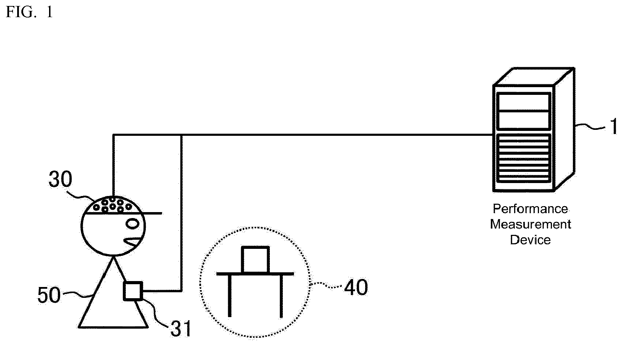

First, an example of where the present invention may be adopted is described using FIG. 1. FIG. 1 schematically illustrates an example of where a performance measurement device 1 according to the embodiment may be adopted. The example illustrated in FIG. 1 assumes a setting where the performance of a worker 50 performing a work step 40 is measured. The work step 40 is among a plurality of work steps included on a production line at a production site. The worker 50 is one example of a worker in the invention, and the work step 40 is an example of a first work step.

The performance measurement device 1 according to the embodiment obtains sensing data (sensing data 121, later described) that be acquired by using one or a plurality of sensors to measure at least one of sensory activity and physical activity while the worker 50 executes a work step 40 from among a plurality of work steps at a production site. A work step performed at a production site may be soldering, fitting cases, inserting parts for insertion, inspection, packaging, or the like. The example in FIG. 1 include an electroencephalogram 30 (EEG 30) and a load cell 31 which are the sensors that acquire the sensing data in the example. The EEG 30 and load cell 31 are each an example of a sensor used in the invention.

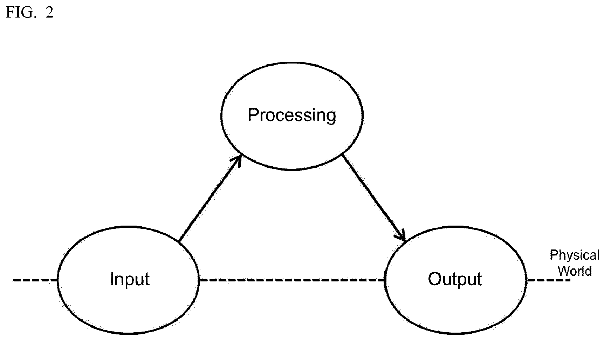

The sensory activity and physical activity measured by the sensors is described here with additional reference to FIG. 2. FIG. 2 schematically depicts a model representing the human cognitive process; as illustrated in FIG. 2, the human cognitive processing of information can be modeled by the following three steps. That is, in the first step, input data is acquired via the input system. An input system is primarily a sensory system. Next, in the second step the processing system (the brain) processes the input data acquired from the input system. In the third step, the results of information processing performed by the processing system is output via the output system. The output system is primarily the musculoskeletal system (and the brain).

According to this model, sensory activity and physical activity can be defined as follows. That is, sensory activity involves using sensory systems such as vision, hearing, and touch as the input system to acquire input data from an object; the processing system processes the input data to recognize attributes of the object such as location, shape, size, and texture; and the processing of information, e.g., decision making, by the processing system is carried out on the basis of the result of what was recognized. Recognition within sensory activity includes spatial recognition and shape recognition of the target. Spatial recognition involves recognizing the attributes concerning a target in a space such as the location, moving speed, or the like. Shape recognition involves recognizing the attributes concerning the form of the target, such as the shape, size, and texture of the target. In contrast, where musculoskeletal system is the system and organs involved in movement of the body, such as the bones, muscles, joints, and nerves, physical activity uses the musculoskeletal system as the output system to move the body on the basis of the above recognition results, or to carry out the above recognition. Thus, physical activity involves moving the body and physically affecting an object or changing the positional relationship with the object.

Humans repeatedly perform this process of cognitive information processing along with the sensory activity and physical activity when accomplishing a work step. As illustrated in FIG. 2, the input system (primarily a sensory system) and the output system (primarily the musculoskeletal system) function as the interface with the physical world while a person is performing a work step. Therefore, the above sensory activity and physical activity regarding a work step may be measured by the behaviors of the input system and the output system. Given the above-mentioned relationship between the input system, the processing system, and the output system, it is possible to indirectly evaluate the quality of information processing performed by the processing system in relation to a work step by using sensors to monitor at least one of sensory activity and physical activity.

That is, the sensing data obtained from measuring at least one of the sensory activity and physical activity using one or a plurality of sensors may be used to indicate the performance in at least one of the sensory activity and physical activity in relation to a work step. The performance of the sensory activity and the physical activity correlates to the results of whether or not a work step can be suitably accomplished. That is, the higher the performance of the sensory activity and the physical activity, the better the quality of information processing performed by the processing system in relation to the work step, and the higher the ability to accomplish the work step (i.e., the work step can be suitably accomplished). Whereas, the lower the performance in the sensory activity and the physical activity, the worse the quality of information processing performed by the processing system in relation to the work step, and the lower the ability to accomplish the work step (i.e., the work step cannot be suitably accomplished). Accordingly, it is possible to evaluate the quality of the above mentioned information processing in relation to the work step; in other words, it is possible to objectively and quantitatively evaluate the level of ability in executing a work step on the basis of sensing data obtained by using one or a plurality sensors to measure at least one of the sensory activity and the physical activity.

The work steps are also achieved through a series of sensory activities and physical activities. Thus, the process of executing a work step may be expressed as a combination of a plurality of types of sensory activity and physical activity. Therefore, it is possible to take a work step as a plurality of basic operations. The basic operations may then be defined from a combination of sensory activity and physical activity. The combination of the same types of sensory activity and physical activity may manifest each time a work step is executed when a person repeatedly executes the same work step. A segment expressing a combination of the same types of activity within executing these work steps may be output as a segment where a common basic operation (i.e. the same type of basic operation) is being performed. Therefore, in order to facilitate extracting the basic operations, repeated executions of the work step is preferable.

In addition, the human cognitive process can be performed for a plurality of cycles while accomplishing a work step. Thus, the basic operations are defined to include the human cognitive process for at least one person for one cycle in order to facilitate identifying the basic operations included in a work step. At this point, the basic operations may be aligned sequentially in a time series, and the results of performing a basic operation taken as input for the next basic operation. In the present embodiment, the basic operations (each of the four basic operations below) contained in a work step are defined as being contained in at least one cycle of a human cognitive information processing process, and are defined by a combination of sensory activity and physical activity.

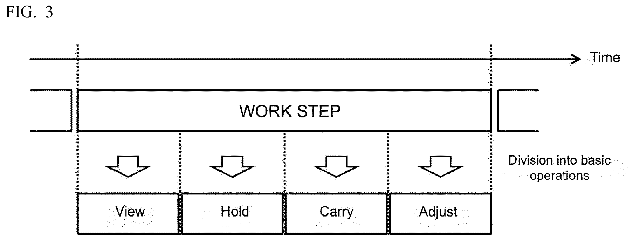

A concrete example of a basic operation is described with reference to FIG. 3. FIG. 3 schematically illustrates an example of a plurality of basic operations making up a work step; As provided by the example illustrated in FIG. 3, a work step (work step 40) is divided into four types of basic operations: "view", "hold", "carry", and "adjust". In the example illustrated in FIG. 3, the basic operations of View, Hold, Carry, and Adjust are aligned in sequence in a time series (along a time axis).

"View", is primarily the activity of recognizing the attributes of an object in the work step. The sensory activity during "View" may involve, for instance, recognizing attributes of the object such as the position, shape, and size through sight or sound. In contrast, the physical activity during "View" may involve moving the body by changing the direction of the gaze, changing the angle of the neck, or pointing to and confirming the object, or the like in order to perform spatial recognition and shape recognition. The sensory activity of "View" may also include recognizing attributes such as the texture of an object via touch, by placing the finger near the object or using the finger to touch the object.

"Hold", is primarily the activity of holding the object in the work step on the basis of the results of "View". The sensory activity during "Hold" may involve, for instance using touch to recognize the texture of the object for the work step while determining a position to hold said object on the basis of spatial recognition and shape recognition of the object via site and touch. In contrast, the physical activity involved in "Hold" may be to move a part of the body such as the hand or the finger on holding the object so that the object does not fall on the basis of the above sensory activity.

"Carry", is primarily the activity of holding the object in the work step 40 on the basis of the results of "View". The sensory activity during "Carry" may involve determining a location of the destination for the object (target position) on the basis of the results of spatial recognition of the object. In contrast, the physical activity during "Carry" may involve moving a part of the body such as the arm, leg, or waist to carry an object being held from the current position to a target position.

"Adjust", is primarily the activity of changing the subsequent condition of the object to a target condition. The sensory activity during "Adjust" may involve, for instance, using sight or touch to recognize a change in the condition of the position, angle, or shape of the object. In contrast, the physical activity during "Adjust" may involve moving a part of the body such as the finger, or the like, while causing changes to the condition of the target object until the object is in the target condition.

The work steps at a production site may be executed via a combination of the above four basic operations. One specific example, is a setting where a worker 50 performs soldering. In this case, "View" involves performing spatial recognition and shape recognition of the object whereon the soldering iron will be placed and that will be soldered; "Hold", means to hold the soldering iron; "Carry", means to carry the soldering iron to the object; and "Adjust" means changing the position and angle of the soldering iron. This series of basic operations may be executed to thereby perform soldering.

Work steps performed at the production site other than soldering may also be executed via a combination of the above four basic operations. However, the type, number, combination, and sequence of the basic operations are not particularly limited to these examples and may be established as appropriate in accordance with the form of implementation. For example, a sequence of basic operations may be defined where "View" is performed after "Adjust". Dividing the work steps at a production site into a plurality of basic operations makes it possible to express a process for executing a work step with a combination of basic operations. It is thus possible to evaluate the above activities of a person during the process of executing a work step. Additionally, the work steps may be divided into a plurality of basic operations whereby different work steps may be expressed with a common metric (a basic operation), and thus the ability of a person in relation to the work steps may be evaluated based on this common metric.

The performance measurement device 1 according to the embodiment analyzes the sensing data obtained from the worker 50. Using the sensing data, the performance measurement device 1 thus computes a performance index indicating the level of performance of the basic operations achieved through the above-mentioned activities.

The performance index (i.e., the level of performance in relation to the basic operations) corresponds to whether or not the basic operations included in a work step 40 can be suitably executed; in other words, the performance index corresponds to the level of ability in executing the basic operations. The performance index can be used to objectively and quantitatively indicate the ability of a worker 50 in each of the basic operations. Accordingly, performance indexes obtained for the basic operations included in a work step 40 can objectively and quantitatively indicate the ability, which is how a worker 50 performs that work step 40. For example, it is possible to indicate in which of the four basic operations contained in a soldering step a worker 50 does well or does poorly.

Additionally, the performance measurement device 1 according to the embodiment compares the performance index needed for each of the basic operations in each of the work steps to suitably accomplish the work steps (hereafter, also referred to as the "required performance index") and the performance index calculated for the worker 50 in each of the basic operations. The performance measurement device 1 also assigns the worker 50 any work step from among the plurality of work steps at a production site on the basis of the comparison results where the performance index calculated for said worker 50 in each of the basic operations satisfies a predetermined criterion in relation to the required performance index for each of the basic operations. Note that here, the "second work step" is an example of "any work step." This "any work step" may coincide with the work step 40, or may be different from the work step 40. Thus, the performance measurement device 1 according to the embodiment uses the results of evaluating the performance index to assign the aforementioned worker a work step for which the worker is suited. The performance measurement device 1 outputs the results of said assignment.

Therefore, the performance measurement device 1 according to the embodiment can objectively and quantitatively measure the ability of a worker 50 to perform a work step 40. The performance measurement device 1 according to the embodiment may be configured to treat each of the workers at a production site as the worker 50, measuring the performance index for each worker and assigning each worker to a work step on the basis of measuring the worker's performance. Thus, the present embodiment can more appropriately assign workers to work steps. Therefore, it is possible to systematically improve or maintain the productivity and efficiency of the production line at the production site. Moreover, even if a worker has no experience with all the work steps at a production site, the embodiment allows for a work step to be assigned to a worker suited thereto on the basis of knowledge obtained through the worker executing any work step (i.e., through the performance index calculated for the basic operations). Therefore, it is possible to reduce the cost associated with optimizing assignments of a worker to a work step.

Note that as above described, the basic operations in the embodiments are defined from a combination of sensory activity and physical activity. Therefore, preferably both sensory activity and physical activity are measured instead of measuring only sensory activity or only physical activity in order to accurately compute the performance index for the basic operations. Therefore, in the embodiment, the performance measurement device 1 can acquire sensing data obtained by using a plurality of sensors to measure the activity of, e.g., the sensory activity and physical activity of the worker 50. More specifically, in the embodiment the EEG 30 can be used to measure the sensory activity of the worker 50. Further, the load cell 31 can be used to measure the physical activity of the worker 50. However, the combination of the plurality of sensors used for measuring the sensory activity and the physical activity of the worker is not limited to this example; the combination of the plurality of sensors may be selected as appropriate in accordance with the form of implementation.

### 2. Example Configuration

### Hardware Configuration

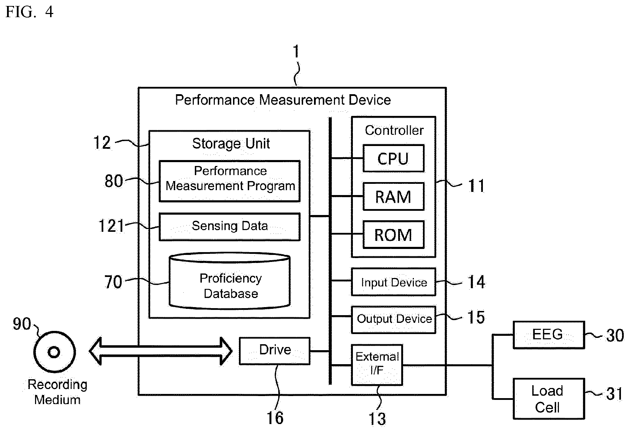

Next an example of the hardware configuration for the performance measurement device 1 according to the embodiment is describes using FIG. 4. FIG. 4 schematically illustrates an example of a hardware configuration for a performance measurement device 1 according to an embodiment.

As illustrated in FIG. 4 the performance measurement device 1 according to the embodiment is a computer with a controller 11, a storage unit 12, an external interface 13, an input device 14, an output device 15, and a drive 16 electrically connected thereto. Note that in FIG. 4 the external interface is noted as an "external I/F".

The controller 11 includes a central processing unit (CPU) that is a hardware processor, random access memory (RAM), and read only memory (ROM), or the like; the controller 11 is configured to process information on the basis of a program and various kinds of data. The storage unit 12 is one example of memory and, for example may be constituted by a hard drive or a solid state drive or the like. The storage unit 12 in the embodiment stores various information such as a performance measurement program 80, sensing data 121, a proficiency database 70, a work database 75, and the like.

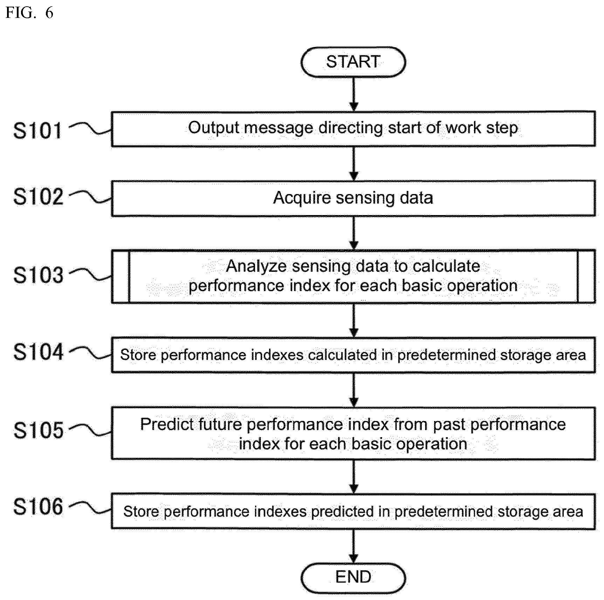

The performance measurement program 80 causes the performance measurement device 1 to process information for measuring the performance index of a worker in each of the basic operations of a work step (FIG. 6, FIG. 14, and FIG. 15). The performance measurement program 80 includes a series of commands for processing information. The sensing data 121 represents the behavior of the worker with respect to at least one of sensory activity and physical activity while the worker executes the work step. The proficiency database 70 stores the performance index in association with the work step and the worker where the performance index was measured by executing the performance measurement program 80. The work database 75 stores information representing the assignment of a worker to a work step. The details are described later.

The external interface 13 may be a universal serial bus (USB) port, or a dedicated port, or the like, and is for connection to an external device. The types and number of the external interface 13 may be selected as appropriate in accordance with the types and number of external devices for connection. The performance measurement device 1 in the embodiment is connected to the EEG 30 and the load cell 31 via the external interface 13.

The EEG 30 may be mounted on the head of the worker 50 and used to measure the amount of brain activity of the worker 50. The amount of brain activity measured pertains primarily to the sensory activity of the worker 50. The load cell 31 may be mounted to a part of the body, such as the hands, or the like of the worker 50 and used to measure the force (load) applied to said part of the body. The force measured pertains primarily to the physical activity of the worker 50. The types of EEG 30 and load cell 31 are not particularly limited and may be selected as appropriate in accordance with the form of implementation.

Accordingly, the sensing data 121 in the embodiment may be obtained by using the EEG 30 and the load cell 31 to measure the behavior, e.g., the sensory activity and physical activity, of the of worker 50, while the worker 50 executes the work step 40. The performance measurement device 1 can obtain sensing data 121 from the EEG 30 and the load cell 31 via the external interface 13.

The input device 14 may be a mouse, keyboard, or the like for input. The output device 15 may be a display, speaker, or the like for output. An operator uses the input device 14 and the output device 15 to operate the performance measurement device 1. The operator may be, for example the worker 50 themselves, or a supervisor who supervises the worker 50.

The drive 16 may be a compact disk drive (CD), a DVD drive, or the like; the drive 16 is for reading a program stored on a recording medium 90. The type of drive may be selected as appropriate in accordance with the type of recording medium 90. The above-mentioned performance measurement program 80 may be stored on such a recording medium 90.

The recording medium 90 may be a medium whereon the information for the program or the like is stored via electrical, magnetic, optical, mechanical, or chemical processes so that a computer, another device, or a machine is capable of reading the information recorded in the program or the like. The performance measurement device 1 may obtain the above mention performance measurement program 80 from the recording medium 90.

FIG. 4 illustrates a CD, or DVD type recording medium as one example of the recording medium 90. However, this does not mean that the type of recording medium 90 is particularly limited to a disc; the recording medium may be of another format; for instance, a flash memory or a semiconductor memory are examples of this non-disc recording medium.

The constituent elements of the specific hardware configuration of the performance measurement device 1 may be omitted, substituted, or added to as appropriate in accordance with the embodiment of the performance measurement device 1; for instance, the controller 11 may contain a plurality of hardware processors. A hardware processor may be configured from a microprocessor, a field-programmable gate array (FPGA), or the like. The storage unit 12 may be configured from the RAM and ROM included in the controller 11. Any one of the external interface 13, input device 14, output device 15, and drive 16 may be omitted. The performance measurement device 1 may include a communication interface that connects to an external device via a network to provide data communication therewith. The EEG 30 and the load cell 31 may be provided with a communication interface; in this case, the performance measurement device 1 may connect to the EEG 30 and the load cell 31 via a network. The performance measurement device 1 may be made up of a plurality of computers. In this case, the hardware configuration of the computers may be identical or may be different. The performance measurement device 1 may be an information processing device designed exclusively for providing a service; beyond this, the performance measurement device 1 may be a general purpose server device, a personal computer (PC) or the like.

### Software Configuration

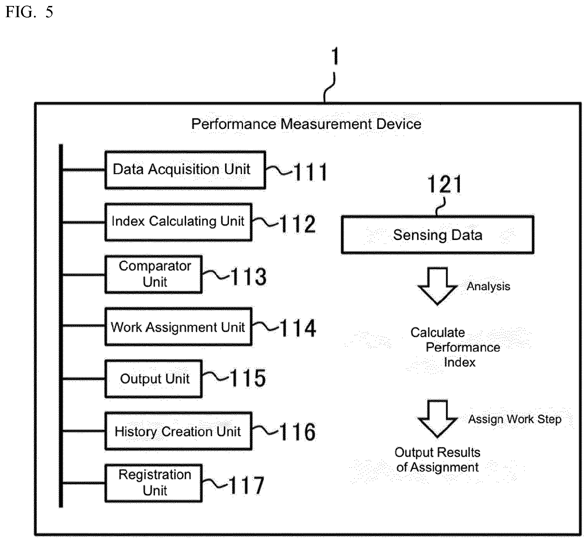

Next, an example of the software configuration for the performance measurement device 1 according to the embodiment is described using FIG. 5. FIG. 5 schematically illustrates an example of a software configuration for the performance measurement device 1 according to an embodiment.

The controller 11 in the performance measurement device 1 expands the performance measurement program 80 stored in the storage unit 12 into RAM. The controller 11 then uses the CPU to compile and execute the performance measurement program 80 expanded to the RAM to control the constituent elements on the basis of a series of commands contained in the performance measurement program 80. Thus, as illustrated in FIG. 5, the performance measurement device 1 operates as a computer provided with the following software modules: a data acquisition unit 111, an index calculating unit 112, a comparator unit 113, a work assignment unit 114, an output unit 115, a history creation unit 116, and a registration unit 117. That is, the controller 11 implements the embodiment with the software modules.

The data acquisition unit 111 obtains sensing data 121 by using one or a plurality of sensors to measure an activity (behavior) related to at least one of sensory activity and physical activity of a worker while said worker executes a first work step from among a plurality of work steps performed at the production site. As above described, a work step is made up of a plurality of basic operations. The index calculating unit 112 analyzes the sensing data 121 acquired, and calculates a performance index for the worker with regard to the basic operations from the sensing data 121. The performance indexes are calculated to indicate a worker's level of performance of the basic operations achieved through the activity (behavior) of the worker.

The comparator unit 113 compares the performance index needed for each of the basic operations within each of the work steps in order to suitably execute the work steps (i.e., a required performance index) and the performance index calculated for the worker in each of the basic operations. The work assignment unit 114 assigns a second work step from the plurality of work steps on the basis of the comparison results where the performance index calculated for the worker in each of the basic operations satisfies a predetermined criterion in relation to the performance index required for each of the basic operations (i.e., the required performance index). The output unit 115 outputs the results of the assignment.

The data acquisition unit 111 may repeatedly acquire sensing data 121 from the worker; further, the index calculating unit 112 may calculate the performance index for worker regarding the basic operations from the sensing data 121 each time acquisition of the sensing data 121 is obtained. The history creation unit 116 collects the performance indexes calculated for a worker in each of the basic operations to create a history of performance indexes for the worker in each of the basic operations. The index calculating unit 112 may predict the future performance index for a worker in each of the basic operations from past performance indexes represented in the history created. The comparator unit 113 may compare the performance index needed for each of the basic operations (i.e., the required performance index) in each of the work steps and a future performance index predicted for the worker in each of the basic operations.

Moreover, the data acquisition unit 111 may acquire sensing data obtained by measuring the activity of a skilled worker skilled operator using one or a plurality of sensors while said skilled operator who is capable of suitably executing third work step from among the plurality of work steps performs said third work step. The index calculating unit 112 analyzes the sensing data acquired from the skilled operator to thereby calculate the performance index of the skilled operator for each of the basic operations. The registration unit 117 registers the performance index calculated for the skilled operator in each of the basic operations in the third work step as the performance index required for each of the basic operations in the third work step (i.e., the required performance index).

The software modules in the performance measurement device 1 are described in detail below with an operation example. Note that all the software modules of the performance measurement device 1 described with the embodiment are examples that may be implemented with a general purpose CPU. However, all or a portion of the above software modules may also be implemented in one or a plurality of dedicated processors. The software configuration of the performance measurement device 1 may be carried out by omitting, substituting or adding software modules as appropriate in accordance with the form of implementation.

### 3. Operation Example

Next, an operation example of the performance measurement device 1 of the embodiment is described. The performance measurement device 1 according to the embodiment is configured to measure the performance of a worker via three modes: a measurement mode, a registration mode, and an assignment mode. The measurement mode computes a performance index for a worker. The registration mode computes the performance index for a skilled operator, and registers the performance index computed for the skilled operator as a required performance index. The assignment mode uses the performance indexes calculated during the measurement mode to assign a worker any work step among the plurality of work steps performed at the production site.

The processing sequence in each mode described below is one example of the performance measurement method of the invention. However, this does not mean that the performance measurement device 1 must always be configured to execute these three modes. The modes may be omitted or modified as appropriate. The processing sequence described below is merely an example, and the processes may be modified where possible. Moreover, the steps in the processing sequence described below may be omitted, substituted, and steps may be added as appropriate in accordance with the form of implementation.

### Measurement Mode

First, an example of a processing sequence during the measurement mode of a performance measurement device 1 according to the embodiment is described using FIG. 6; FIG. 6 is a flowchart illustrating an example of a processing sequence during the measurement mode of a performance measurement device 1 according to the embodiment.

### Step S101

In step S101, the controller 11 outputs a message directing the worker 50 to execute a work step 40 among the work steps the plurality of work steps at the production site. The destination and method of outputting the message is not particularly limited and may be selected as appropriate in accordance with the form of implementation.

As a concrete example, the controller 11 may output the message via the output device 15 or an output device (not shown) located near the worker 50, for example, to direct the worker 50 to execute the work step 40. The types of work steps 40 directed for execution are not particularly limited and may be selected as appropriate in accordance with the form of implementation. The work step 40 for execution may be soldering, fitting cases, inserting parts for insertion, inspection, packaging, or the like. Once the message is output directing execution of the work step 40, the controller 11 continues to performing the next step S102.

### Step S102

The data acquisition unit 111 obtains sensing data 121 by using one or a plurality of sensors to measure an activity related to at least one of sensory activity and physical activity for a worker while said worker executes a first work step from among a plurality of work steps at the production site.

In the embodiment, an EEG 30 and a load cell 31 are attached to the worker 50, and the performance measurement device 1 is connected to the EEG 30 and the load cell 31 via the external interface 13. Therefore, the sensing data 121 in the embodiment may be obtained by using the EEG 30 and the load cell 31 to measure the activity relating to the sensory activity and physical activity of the of worker 50, while the worker 50 executes the work step 40 on the production line. That is, the sensing data 121 according to the embodiment is made up of two types of data: a first measurement data containing the amount of brain activity measured by the EEG 30; and a second measurement data containing the load measured by the load cell 31. The sensing data 121 may include the data obtained while the worker 50 performs the work step 40 one time, or may include the data obtained while the worker 50 performs the work step multiple times. In the embodiment, the controller 11 acquires said sensing data 121 from the EEG 30 and the load cell 31 via the external interface 13. The work step 40 contains the above mentioned four types of basic operations. The basic operations of "View", "Hold", "Carry", "Adjust" are defined to include the human cognitive process for at least one cycle; however, the composition and method of acquiring the sensing data 121 are not particularly limited to these examples and may be selected as appropriate for the form of implementation. Once the sensing data 121 is acquired, the controller 11 moves to performing the next step S103.

### Step S103

In step S103 the controller 11 acts as the index calculating unit 112 and analyzes the sensing data 121 acquired to compute a performance index indicating the level of performance of the basic operations achieved through the activity (behavior) of the worker. In this embodiment, the controller 11 analyzes the sensing data 121 acquired from the worker 50 to compute a performance index for each of the basic operations included in the work step 40.

The sensing data 121 acquired represents the performance for at least one of the sensory activity and the physical activity in relation to the basic operations contained in a work step. (In this embodiment, the sensing data 121 acquired represents both types of performance). Appropriately accomplishing a work step means that the basic operations achieved as a result of the sensory activity and the physical activity are executed with high precision. Therefore, performance in relation to the basic operations can be evaluated on the basis of the precision in executing the basic operations.

More specifically, the ability to suitably accomplish a work step means the ability to execute the basic operations in the correct order at a suitable speed. Additionally, when the work step is attempted repeatedly, the worker has a greater ability to accomplish the work step, and there are fewer variations during an attempt to execute the basic operations; in other words, the worker is able to execute the basic operations identically at each attempt. Therefore, the precision in executing the basic operations may be represented by the correctness, stability, speed, rhythm, and the like of executing the basic operations.

The correctness is a metric indicating a measure of whether or not the basic operations were executed in the correct sequence when attempting the work step once. The stability is a metric indicating a measure of whether or not the basic operations are executed with a uniform procedure for each attempt when the work step is attempted a plurality of times. The speed is a metric indicating a measure of the length of time spent on the basic operations and the overlap with a neighboring basic operation when attempting the work step once. The rhythm is a metric indicating a measure of whether or not a fixed time is spent on the basic operations during each attempt in the case that the work step is attempted a plurality of times. Given these four metrics of correctness, stability, speed, and rhythm, it is possible to appropriately evaluate the performance of a worker with respect to the basic operations.

In this embodiment, the controller 11 uses these four metrics to analyze the sensing data 121 and compute a performance index for each of the basic operations in step S103. However, these four metrics are merely examples of the metrics used to evaluate the precision of executing the basic operations. The metrics used to evaluate the precision of executing the basic operations are not limited to these examples and may be determined as appropriate in accordance with the form of implementation.

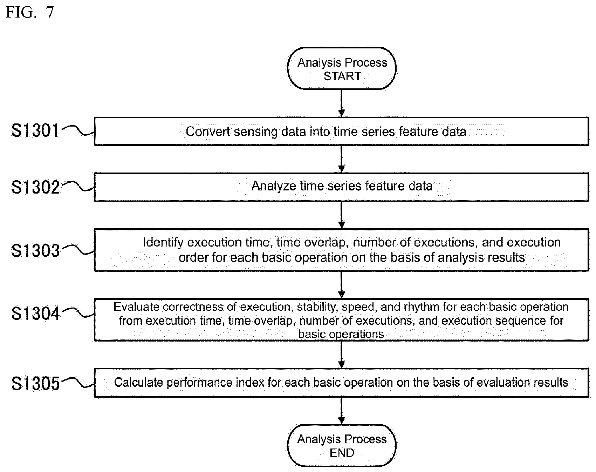

Here, step S103 according to the embodiment is described in detail using FIG. 7. FIG. 7 schematically illustrates an example of a processing sequence that analyzes sensing data 121; the processing in step S103 according to the embodiment includes the processing in the following steps S1301 through S1305.

### Step S1301

In step S1301, the controller 11 converts the sensing data 121 to time series feature data. Any well-known technique maybe adopted for the conversion process as appropriate.

As an example of the conversion process, the controller 11 may first divide sensing data 121 into fixed intervals to partition the sensing data 121 into a plurality of frames. If not needed, the process of partitioning into frames may be omitted. Next, the controller 11 executes a predetermined computation process on a portion of data in each frame to calculate the features within each frame. The controller 11 then plots the features calculated along a time axis. With this series of processes, the controller 11 converts the sensing data 121 into time series feature data. Once the sensing data 121 is converted, the controller 11 moves to performing the next step S1302.

Note that the features may be measurement values for force, motion, point of gaze (gaze direction), or brain waves; for example, the features may be the amplitude, maximum (peak value), minimum, mean, variance, standard deviation, instantaneous value, or the like. The features may be expressed in a probability distribution along a time axis. One or a plurality of features may be adopted as the points used to generate the time series data. That is, the sensing data 121 may be converted into time series data for one or a plurality of features.

Additionally, a feature may be selected that can be commonly monitored among work steps when evaluating different work steps using a common metric (i.e., a basic operation). For example, assume that the peak force value is uniquely expressed in the basic operations contained in a work step T and a work step U. That is, assume that the peak force value that appears within the sensing data can be analyzed during step S1302 (later described). In this case, the peak force value is adopted and used to generate the time series data.

### Step S1302 and S1303

In step S1302, the controller 11 analyzes the time series feature data to estimate the time segments during which the worker executes the basic operations along a time axis. Next, the controller identifies the execution time, time overlap, number of executions, and execution order for each basic operation in step S1303 on the basis of the analysis results.

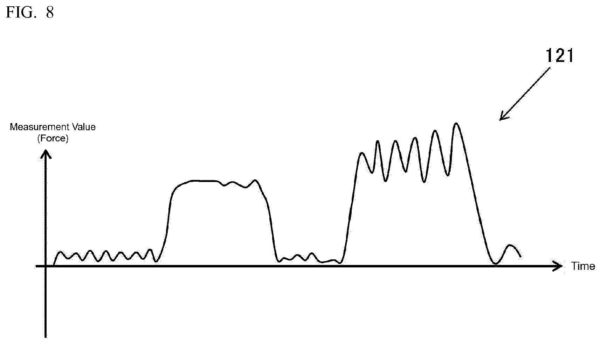

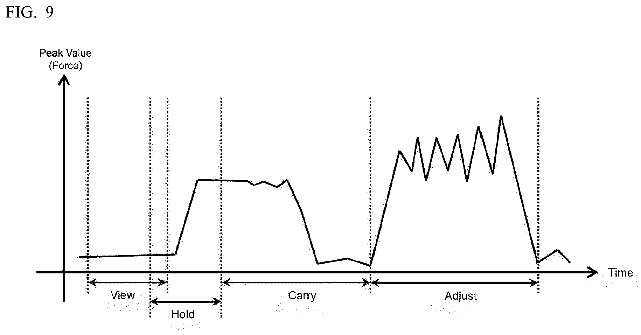

The processes in steps S1301 through S1303 are described in detail using FIG. 8 and FIG. 9. FIG. 8 schematically illustrates an example of the sensing data 121; and FIG. 9 schematically illustrates an example of time series feature data. The sensing data 121 in the embodiment is made up of a first measurement data containing the amount of brain activity measured by the EEG 30 and a second measurement data containing the load measured by the load cell 31. However, in steps S1301 through S1303, the first measurement data and the second measurement data are processed identically. Therefore, for convenience, the description of the first measurement data below is provided assuming that the first measurement data is processed identically to the second measurement data.

As illustrated in FIG. 8, the measurement values for load (force) measured by the load cell 31 are aligned along a time axis in the sensing data 121 according to the embodiment. Adopting the peak force value as a feature, and applying this to the sensing data 121 in the above-mentioned step S1301 results in the time series data illustrated in FIG. 9. Here, the peak force value is lined up along a time axis. Applying the processing of the above step S1302 to the time series data makes it possible to estimate which time in the time series data corresponds to what basic operation. (That is, it is possible to estimate which of the four basic operations in the work step 40 that a worker 50 executing at a given time within the time series data). In other words, it is possible to estimate a correspondence relationship between the time segments in the time series data and the basic operations. Hereby, in step S1303 it is possible to identify execution time, time overlap, number of executions, and execution order of the basic operations within the processes the worker 50 performs to accomplish the work step 40 as illustrated in FIG. 9.

More specifically, the length of a time segment for each of the basic operations and the overlap with a neighboring time segment represents the length of time spent executing the basic operations (the execution time) and the amount of overlap with a neighboring basic operation (time overlap). In the example illustrated in FIG. 9, the execution times for "Carry" and "Adjust" are longer than the execution times for "View" and "Hold". "View" and "Hold" have some time overlap. Additionally, the number of time segments corresponding to each of the basic operations indicates the number of times the basic operations are executed (the number of executions). In the example in FIG. 9, each of the basic operations are executed once. Additionally, the lineup (sequence) of time segments corresponding to each of the basic operations indicates the sequence in which the basic operations are executed (the execution order). In the example in FIG. 9, the basic operations: "View", "Hold", "Carry", and "Adjust" are executed in that order. On thus identifying the execution time, the time overlap, the number of executions, and the execution order for the basic operations, the controller 11 continues to processing in the next step S1304.

The techniques for analyzing time series data is not particularly limited as long as it is possible to identify time segments in which the basic operations are executed within the time series data; the analysis technique may be selected is appropriate in accordance with the form of implementation. Any known methods of clustering may be used to analyze the time series data; for example, a state transition efficiency model, Bayes model, Markov model, hidden Markov model, multi-class identification model, kernel function, dynamic time warping, or the like may be adopted.

Additionally, a supervised learning model may be used on the time series data, where the supervised learning model learns to estimate the time segments for the basic operations via machine learning. The machine learning process may use a data set made up of a combination of time series feature data taken as a sample and correct data indicating the time segments for a basic operation in the sample. The sample is training data. The learning model may be constituted by a neural network, a support vector machine, or the like. The learning model may be trained using a known learning algorithm such as back propagation or the like, so that the learning model outputs the correct data corresponding to a sample when a sample is entered. Therefore, on receiving time series feature data, a supervised learning model is capable of outputting results estimating the time segments for each of the basic operations in the time series data entered.

### Step S1304

In step S1304, the controller 11 evaluates the correctness, stability, speed, and rhythm of executing each of the basic operations on the basis of the execution time, time overlap, number of executions, and execution order identified for each of the basic operations in step S1303.

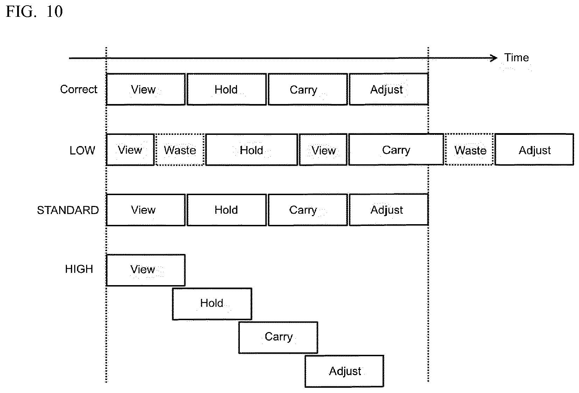

The processes a person uses to master a work step are described using FIG. 10. FIG. 10 schematically illustrates an example of a process of a human mastering a work step. In the example in FIG. 10, a work step contains four basic operations: "View", "Hold", "Carry", and "Adjust". It is assumed that performing these basic operations in this order in a suitable time leads to suitably accomplishing the work step ("correct", FIG. 10).

A person who has not mastered the work step, i.e., a beginner with a low ability to accomplish the work step, cannot execute the basic operations in the correct order and in a suitable time. Therefore, the beginner has a low precision in executing the work step and the beginner cannot complete the work step in a standard time at a standard quality. Accordingly, as illustrated by the "Low Level" in FIG. 10, the processes executed by a beginner to accomplish the work step is such that the time spent for a basic operation is longer than the suitable time; or the time spent on the basic operation is extremely short (i.e., insufficient time is used to execute the basic operation) therefore, the basic operation has to be repeatedly performed; or, there is time not used on any basic operation, or is wasted; or, there is an error in the order in which the basic operations are performed.

As a beginner starts to master a work step, the beginner gradually wastes less time in the process of executing the work step, and is able to seamlessly perform the basic operations in the correct order within a suitable time. Hereby, the beginner becomes a skilled operator with an ability at a "Standard Level" illustrated in FIG. 10. That is, a skilled operator at a standard level is able to execute the basic operations seamlessly, in the correct order, and within a suitable time; therefore, the skilled operator is able to complete the work step within the standard time at a standard quality. When the skilled operator at a standard level further masters the work step, the skilled operator is able to execute the basic operations even more seamlessly and in a shorter time. Thus, as at the "High Level" illustrated in FIG. 10, there is a time overlap between neighboring basic operations, and less time is spent on each of the basic operations.

The controller 11 evaluates the correctness, stability, speed, and rhythm of execution of each of the basic operations on the basis of the execution time, time overlap, number of executions, and execution order for the basic operations to estimate at which level a worker belongs, i.e., from low to high. In other words, the more a worker accomplishes a work step repeatedly at the high level, the higher the controller 11 evaluates the correctness, stability, speed, and rhythm of execution of the basic operations by the worker; and in accordance with this the controller 11 calculates a high performance index for each of the basic operations in step S1305 (later described). In contrast, the more a worker accomplishes a work step repeatedly at the low level, the lower the controller 11 evaluates the correctness, stability, speed, and rhythm of execution of the basic operations by the target operator; and in accordance with this the controller 11 calculates a low performance index for each of the basic operations in step S1305 (later described). The method of evaluation for each of the metrics is described below. As above described, the standard level is treated as the level of a skilled operator, and a level where the skilled operator demonstrates further mastery is treated as a high level. However, the levels established for a skilled operator are not limited to these kinds of examples; for instance, the high level may be treated as the only level for the skilled operator.

### (A) Correctness

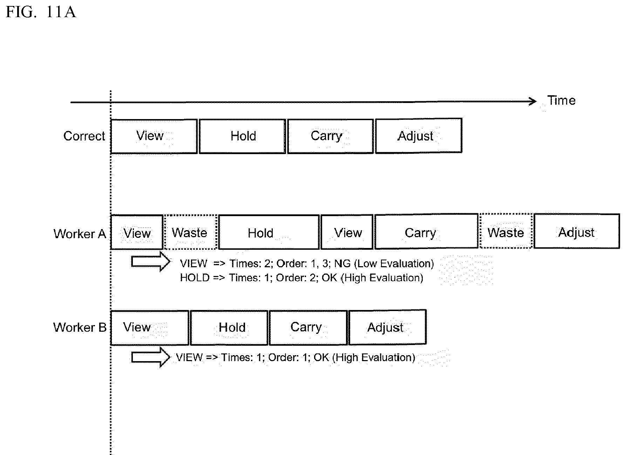

First, the method for evaluating the correctness of executing a basic operation is described with reference to FIG. 11A. FIG. 11A schematically illustrates an example of a method for evaluating the correctness of an execution of a basic operation in the work step illustrated in FIG. 10. The correctness is a metric indicating a measure of whether or not the basic operations were executed in the correct sequence. Therefore, the controller 11 evaluates the correctness of executing each of the basic operations on the basis of the number of executions and the execution order of the basic operations identified in step S1303.

For example, in FIG. 11A, a worker A executes the basic operation "View" two times, with each "View" basic operation executed first and third. In contrast, when the basic operations are executed in the correct order, the basic operation "View" is executed first, only once. Therefore, the worker A, makes a mistake in the number of times and the order for executing "View"; thus, the controller 11 evaluates the correctness of executing "View" for the worker A as low. Whereas, worker B executes the basic operation "View" first, and only once. Therefore, the worker B, makes executes the basic operation "View" in the correct order for the correct number of times; thus, the controller 11 evaluates the correctness of executing "View" for the worker B as high.

That is, the controller 11 evaluates the correctness of execution of the basic operations by the worker as lower with distance to the number of executions and the execution order of the basic operations executed by the worker compared to the number of executions and the execution order of the basic operations when suitably accomplishing the work step. Whereas, the closer number of executions and the execution order of the basic operations executed by the worker compared to the number of executions and the execution order of the basic operations when suitably accomplishing the work step, the higher the controller 11 evaluates the correctness of execution of the basic operations by the worker. In the example in FIG. 11A, while the worker A made errors with respect to the number of executions and the execution order for "View", the worker A has the correct number of executions and correct order for "Hold". Therefore, the controller 11 evaluates a high level of correctness for the execution of "Hold" by worker A.

### (B) Stability

Next, the method for evaluating the stability of executing a basic operation is described. The stability is a metric indicating a measure of whether or not the basic operations are executed with a uniform procedure for each attempt when the work step is attempted a plurality of times. The controller 11 thus evaluates the correctness of executing each of the basic operations during an attempt on the basis of the number of executions and the execution order of each of the basic operations. Thereafter, the controller 11 evaluates the stability of executing each of the basic operations on the basis of the variations in the correctness during the attempt. The variation may be expressed via known mathematical methods such as variance, and standard deviation.

For example, if the worker repeatedly executes each of the basic operations the correct number of times and in the correct order as the worker B in FIG. 11A, then the controller 11 evaluates the worker as executing the basic operations with a high stability. Whereas, the larger the variation in the number of executions and the execution order of the basic operations at each attempt, the lower the controller 11 evaluates the stability of execution for the worker executing the basic operations.

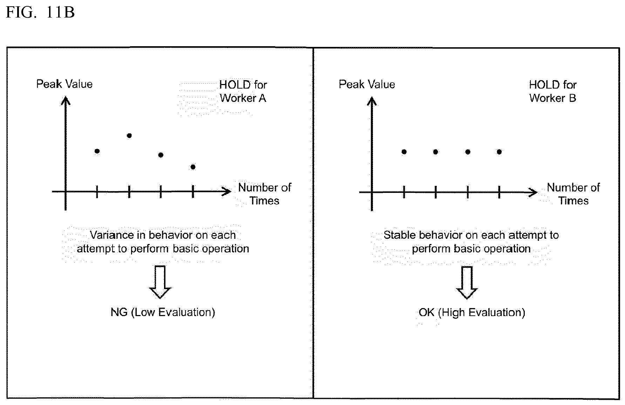

As illustrated in FIG. 11B, the stability of each of the basic operations can be evaluated on the basis of the behavior of the worker identified from the sensing data 121 or the time series feature data. FIG. 11B schematically illustrates an example of a method for evaluating the stability of an execution of the basic operations; the graph for each of the workers (A, B) in FIG. 11B are examples of the peak force value when the workers attempt to "Hold" as identified from the time series feature data.

As above described, the higher the ability of a worker to accomplish a work step, the more the worker is capable of executing each of the basic operations in the same manner during an attempt. Accordingly, the larger the variation in the behavior when accomplishing the basic operations, the lower the controller 11 evaluates the stability of execution by the worker executing the basic operations. In contrast, the smaller the variation in behavior when accomplishing the basic operations, the higher the controller 11 evaluates the stability of execution by the worker executing the basic operations.

In the example in FIG. 11B, there is some variation in the peak force value when the worker A executes "Hold" during one attempt. Therefore, the controller 11 evaluates a low level of stability for the execution of "Hold" by worker A. In contrast, the peak force value when the worker B executes "Hold" during one attempt is constant. Therefore, the controller 11 evaluates a high level of stability for the execution of "Hold" by worker B.

The number of times a work step is attempted in order to evaluate the stability may be established as appropriate in accordance with the form of implementation. The number of attempts may be over a predetermined time, such as over one day, or over one hour, or the like. Given that the work step according to the embodiment is also a work step 40 in a stage on a production line, the number of attempts may be established on the basis of the number of individual products that will be produced via the work step 40.

### (C) Speed

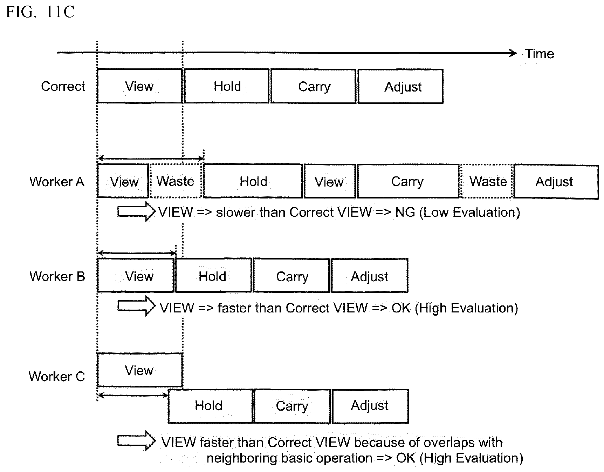

Next, the method for evaluating the speed of executing a basic operation is described with reference to FIG. 11C. FIG. 11C schematically illustrates an example of a method for evaluating the speed of an execution of a basic operation in the work step illustrated in FIG. 10. The speed is a metric indicating a measure of the length of time spent on the basic operations and the overlap with a neighboring basic operation. Therefore, the controller 11 evaluates the speed of executing each of the basic operations on the basis of the execution time and the time overlap for the basic operations identified in step S1303.

For instance, given that the worker A performs some wasted movements between "View" and "Hold", this time is added to the basic operation of "View" making the amount of time spent longer than the suitable time. Therefore, the controller 11 evaluates the speed of execution of the "View" by the worker A as slow. Whereas, the amount of time the worker B spends on the basic operation "View" is shorter than the suitable time. Therefore, the controller 11 evaluates the speed of execution of the "View" by the worker B as fast. Similarly, execution of a portion of the "View" and "Hold" operations by the worker C overlap; therefore, the time worker C spends on the basic operation "View" is less than the suitable time. Therefore, the controller 11 evaluates the speed of execution of the "View" by the worker C as fast.

That is, the shorter the time spent on the basic operations compared to the suitable time on the basis of the execution time and the time overlap in the basic operations executed by the worker, the faster the controller 11 evaluates the speed of execution of the worker for each of the basic operations. Whereas, the longer the time spent on the basic operations compared to the suitable time, the slower the controller 11 evaluates the speed of execution of the worker for each of the basic operations.

Basically, the faster the speed of executing the basic operations, the higher the controller 11 evaluates said speed. However, it is possible that the basic operation was insufficiently executed if the speed of executing the basic operations is extremely faster than the suitable speed. Therefore, the controller 11 may determine whether or not the difference between the speed at which the worker executes the basic operations and the suitable speed of execution exceeds a given threshold, when the speed at which the worker executes the basic operations is faster than the suitable speed of execution. If the difference in speed does not exceed the threshold, the controller determines to evaluate the speed as high level. On the other hand, if the difference in speed exceeds the threshold, the controller evaluates the speed as low level.

### (D) Rhythm

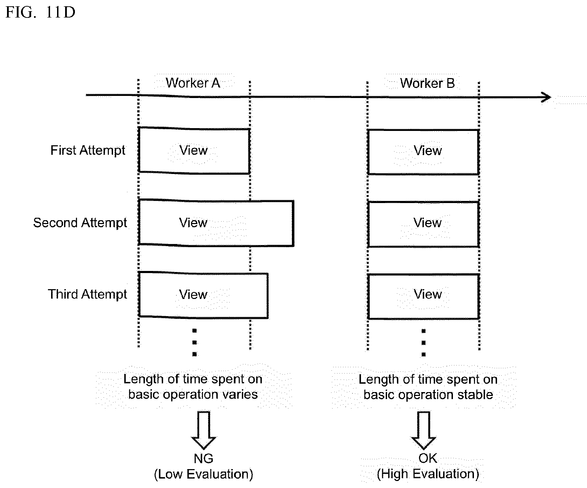

Next, the method for evaluating the rhythm of executing a basic operation is described with reference to FIG. 11D. FIG. 11D schematically illustrates an example of a method for evaluating the rhythm in an execution of a basic operation. The rhythm is a metric indicating a measure of whether or not essentially a fixed time is spent on the basic operations during each attempt in the case that the work step is attempted a plurality of times. Therefore, the controller 11 evaluates the speed of executing each of the basic operations during an attempt on the basis of the execution time and the time overlap of each of the basic operations. Thereafter, the controller 11 evaluates the rhythm of executing each of the basic operations on the basis of the variations in the speed during the attempt.

For instance, there are variations in the lengths of time the worker A spends on "View" in FIG. 11D. Therefore, the controller 11 evaluates the rhythm of execution of the "View" by the worker A as bad. On the other hand, the worker B spends a fixed length of time on "View". Therefore, the controller 11 evaluates the rhythm of execution of the "View" by the worker B as good.

In other words, the greater the variation in the speed of execution of the basic operations (i.e., the length of time spent) when the worker attempts a work step a plurality of times, the controller 11 evaluates the worker as having worse rhythm when executing each of the basic operations. In contrast, the smaller the variation in the speed of execution of the basic operations (i.e., the length of time spent), the controller 11 evaluates the worker as having a better rhythm when executing each of the basic operations.

As above described, the controller 11 evaluates the correctness, stability, speed, and rhythm of executing each of the basic operations on the basis of the execution time, time overlap, number of executions, and execution order identified for each of the basic operations in step S1303. Once each of the basic operations are evaluated, the controller 11 moves to performing the next step S1305.

Note that the correct order and the suitable time (speed) for each of the basic operations included in a work step may be determined as appropriate in accordance with the form of implementation. For example, the correct order and suitable time for each of the basic operations may be presented in advance, or may be established from an operator input. Additionally, the correct order and suitable time for each of the basic operations may be specified on the basis of the behavior of a skilled operator while the skilled operator executes the work step; for instance, during a registration mode (later described), the controller 11 may specify the correct order and the suitable time for each of the basic operations from the sensing data 121 acquired from a skilled operator.

### Step S1305

In step S1305, the controller calculates a performance index for each of the basic operations in accordance with the results of the evaluation in step S1304. The greater the degree of correctness, the greater the stability, the faster the speed, and the better the rhythm of executing a basic operation (i.e., each basic operation has a high evaluation), the higher the value the controller 11 calculates for the performance index for the basic operation. In contrast, the lower the degree of correctness, the lower the degree of stability, the slower the speed, and the worse the rhythm of executing a basic operation (i.e., each basic operation has low evaluations), the lower the value the controller 11 calculates for the performance index for the basic operation.

For example, exemplary behavior for accomplishing each of the basic operations in the correct order and within a suitable time is given a performance index that is a standard value (e.g., the behavior labels "Correct", FIG. 10). This standard value may be applied in advance, or may be established based on input from an operator or the like. The controller 11 compares the degree of evaluations for the worker executing each of the basic operations with the preliminary applied exemplary behavior; further, the controller 11 may calculate a performance index for each of the basic operations from the standard value in accordance with the comparison. That is, the higher the evaluation for each of the basic operations executed by the worker compared to the exemplary behavior, the same or the higher the controller 11 computes calculates a performance index for each of the basic operations. Whereas, the lower the evaluation for each of the basic operations executed by the worker compared to the exemplary behavior, the lower the controller 11 calculates a performance index for each of the basic operations. A computational model such as a linear regression model, covariance structure analysis, or multiple regression analysis in calculating the performance indexes. The performance index may be computed on the basis of comparison with a threshold obtained by normalizing all the data. The exemplary behavior may be applied via input from an operator or the like, or applied from the actions of a skilled operator.

Note that when using a common metric (basic operation) between different work steps to compare the ability of a worker to accomplish a work step, the standard value for each of the performance indexes is established in the embodiment in accordance with the difficulty of the sensory activity and the physical activity required for each of the basic operations.

For example assume the existence of two work steps: work step X and work step Y. Also assume that the sensory activity and the physical activity required for "View" in the work step X is to touch the object with the finger to recognize the state (one-dimensional information) of an object on the basis of the sensation transferred from the object, as if performing an external inspection of a product or the like. In contrast, the sensory activity and the physical activity required for "View" in the work step Y is to touch an object to be connect to another object with the finger, and to recognize the state (two-dimensional information) of the object from the sensation transferred to the finger from the object as if performing an examination of the object by touch.

In this case, clearly there is a greater difficulty in the sensory activity and the physical activity required to "View" in the work step Y than the difficulty in the sensory activity and the physical activity required to "View" in the work step X. At this point, the performance index calculated for "View" when the work step X is accomplished cannot be simply compared to the performance index calculated for "View" when the work step Y is accomplished if to "View" in the work step X and to "View" in the work step Y are each given the same reference value standard value. In other words, a worker capable of executing "View" in work step X with a high performance index is not necessarily capable of executing "View" in work step Y with the same high performance index.

Therefore, as above described, the standard value for each of the performance indexes is established in accordance with the difficulty of the sensory activity and the physical activity required for each of the basic operations included in each of the work steps when using a common metric (basic operation) between different work steps to compare the ability of a worker to accomplish work steps. For example, a determination rule may be established for determining the difficulty of the sensory activity and the physical activity; the standard value may then be determined for a performance index for each of the basic operations established for the work steps on the basis of the established determination rule.

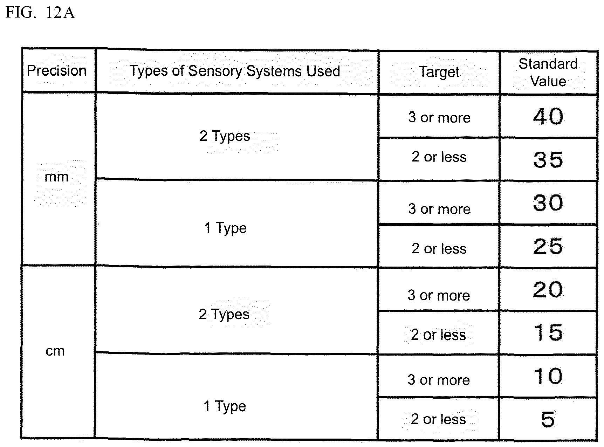

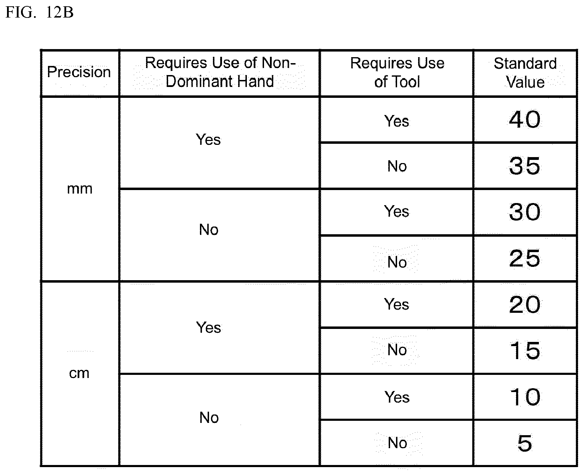

Here, examples of determination rules are described using FIG. 12A and FIG. 12B. FIG. 12A illustrates an example of the relationship between the difficulty of sensory activity and a standard value for a performance index; and FIG. 12B illustrates an example of the relationship between the difficulty of a physical activity and a standard value for a performance index.

In the example of the determination rule in FIG. 12A, the difficulty of the sensory activity is evaluated on the basis of three parameters: the precision of the senses, the types of sensory systems used, and the number of sensory objects. That is, the higher the precision of the senses, the greater the types of sensory systems used, and the greater the number of sensory objects required, the higher the difficulty of the sensory activity. Therefore, in the determination rule depicted in FIG. 12A when a basic operation requires the precision in millimeters (mm), use of two types of sensory systems, and three or more sensory objects, the standard value for the performance index is established as the highest value of "40". In contrast, in the determination rule depicted in FIG. 12A, when a basic operation requires the precision in centimeters (cm), use of one type of sensory system, and no more than two sensory objects, the standard value for the performance index is established as the lowest value of "5 ". In the determination rule depicted in FIG. 12A, the difficulty of the sensory activity can be evaluated at eight levels on the basis of the above three parameters, with the standard value established at equal intervals in accordance with the levels of difficulty.

In the determination rule depicted in FIG. 12B, the difficulty of the physical activity is evaluated on the basis of three parameters: the precision of the physical movement, whether or not the non-dominant hand is used, and whether or not a tool is used. That is, the higher the precision of the physical movement, the use of the non-dominant hand, and the use of the tool required the higher the difficulty of the physical activity. Therefore, in the determination rule depicted in FIG. 12B, a basic operation requiring the precision of physical movement in millimeters (mm), the use of the non-dominant hand, and the use of the tool is given the highest value "40" as the standard value for the performance index. In contrast, in the determination rule depicted in FIG. 12B, a basic operation requiring the precision of physical movement in centimeters (cm), no use of the non-dominant hand, and no use of the tool is given the lowest value "5" as the standard value for the performance index. Similar to the determination rule depicted in FIG. 12A, in the determination rule in FIG. 12B, the difficulty of the physical activity can be evaluated at eight levels on the basis of the above three parameters, with the standard value established at equal intervals in accordance with the levels of difficulty.

The controller 11 uses the determination rules in FIG. 12A and FIG. 12B to thereby determine a standard value for a performance index established for each of the basic operations within each of the work steps in accordance with the difficulty for each of the basic operations. For example, when both determination rules are used, the controller 11 may evaluate the difficulty for each of the sensory activity and the physical activity on the basis of each determination rule, and total the standard value derived from each of the determination rules to thereby establish a standard value for the performance index for each of the basic operations in accordance with the difficulty of each of the basic operations. The controller 11 uses the standard value established for the each of the basic operations included in each of the work steps to calculate performance indexes for each of the basic operations in accordance with the results of the evaluation from step S1304. It is thus possible to compare the ability for a worker to accomplish each of the work steps using a common metric (a basic operation) between different work steps.

However, the parameters for evaluating the difficulty of the sensory activity and the physical activity is not limited to these examples and maybe established as appropriate in accordance with the form of implementation. The parameters other than those given above may be used for evaluating the difficulty of sensory activity, and may be, for example, the recognition object (one-dimensional information or two-dimensional information), the recognition location (e.g., recognizing the external state or the internal state of the object), or the like. In contrast, the parameters other than those given above may be used for evaluating the difficulty of physical activity such as the number of body parts that are moved, the movement time, or the like. Additionally, the correspondence relationship between the difficulty of the sensory activity and the physical activity and the standard value for the performance index is not limited to the examples given in FIG. 12A and FIG. 12B; the correspondence relationship may be established as appropriate in accordance with the evaluation parameters.

The controller 11 uses this series of processes in the above step S1301 through S1305 to analyze the sensing data 121 to thereby calculate a performance index for each of the basic operations. In this embodiment, the controller 11 analyzes the sensing data 121 acquired from the worker 50 to compute a performance index for each of the basic operations included in the work step 40. Once the performance index is computed for each of the basic operations, the controller 11 continues to performing the next step S104.

The series of computational processes in steps S1301 through S1305 may be modeled. That is, the controller 11 may directly derive a performance index for each of the basic operations from the sensing data 121 using a computational model. A supervised learning model may serve as this computational model, where the supervised learning model trains on the ability to derive a performance index for each of the basic operations from the sensing data 121 through machine learning. The machine learning process may use a data set made up of a combination of sample sensing data (training data) and correct data representing the performance index derived from the sample for each of the basic operations. The learning model may be constituted by a neural network, a support vector machine, or the like. The learning model may be trained using a known learning algorithm such as back propagation or the like, so that the learning model outputs the correct data corresponding to a sample when a sample is entered. Hereby, on receiving the sensing data 121, the supervised learning model is capable of outputting a performance index for each of the basic operations as derived from the sensing data 121 that is entered.

### Step S104

Returning to FIG. 6, in step S104, the controller 11 acts as the history creation unit 116 and associates the worker 50, the work step 40, and the performance index for each of the basic operations calculated in step S103, and stores these in a predetermined storage area.

The predetermined storage area and the format of the data is not particularly limited and may be selected as appropriate in accordance with the form of implementation. The predetermined storage area may be the internal RAM of the controller 11, the storage unit 12, an external storage device, a storage medium, or a combination of these. The storage medium may be, for example a CD or DVD or the like, and the controller 11 may store data to the storage medium via the drive 16. The external storage device, may be for instance, an externally installed storage device connected via the external interface 13. In this case, the controller 11 may store data on the externally installed storage device via the external interface 13. If the performance measurement device 1 can be connected to a network, the external storage device may be a data server such as a network attached storage (NAS) device. In this case, the controller 11 may store data to the data server via the network. In this embodiment, the storage unit 12 maintains the proficiency database 70. The controller 11 thus associates and stores the worker 50, the work step 40, and a performance index calculated for each basic operation in the proficiency database 70 on the storage unit 12.

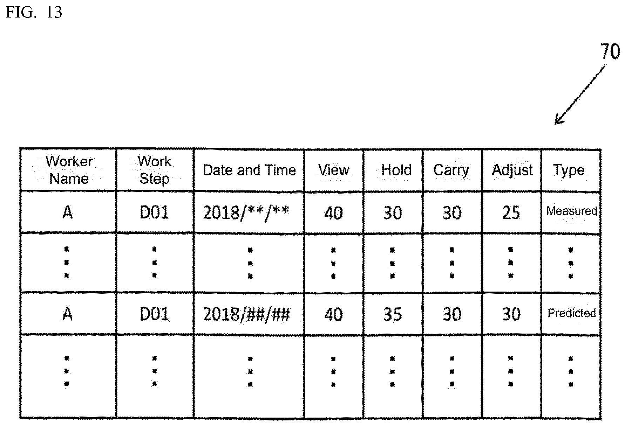

An example of the proficiency database 70 is described using FIG. 13. FIG. 13 schematically illustrates an example of a proficiency database 70 according to the embodiment. The proficiency database 70 stores the performance index for each worker. In the example in FIG. 13, the proficiency database 70 is expressed in table form where each record (row data) includes the fields: Worker Name, Work Step, Date and Time, View, Hold, Carry, Adjust, and Format.

The worker name field stores information for identifying the worker 50. The work step field stores information for identifying the work step 40. The date-and-time field stores information representing the date and time the performance index was measured. The View, Hold, Carry, and Adjust fields store the performance index computed for each of these basic operations. The type field stores information indicating the method by which a performance index is derived. In the example in FIG. 13, "Measured" indicates that the performance index is derived directly from the sensing data 121 acquired in step S102. Whereas, "Predicted" indicates that a future performance index was derived from past performance indexes in step S106 (later described). However, the data format and the fields in the proficiency database 70 are not limited to this type of example and may be selected is appropriate in accordance with the form of implementation.

In step S104, the controller 11 creates a record in the proficiency database 70. The controller 11 stores information identifying the worker 50, information identifying the work step 40, information representing the date and time, the performance indexes calculated in step S103 for each of the basic operations in fields of the record created, and information indicating how the performance indexes were derived (in this step S104, "Measured"). With a worker 50, a work step 40, and the performance indexes computed for the basic operations thusly associated and stored in the proficiency database 70, the controller 11 continues to the processing in the next step S105.

The controller repeats the processes in the above steps S102 through S104 in this embodiment. That is, the controller 11 repeats acquiring the sensing data 121 for the worker 50 through the step S102; each time the sensing data 121 is acquired the controller 11 also calculates the performance index for the worker 50 regarding the basic operations through the step S103. Through step S104 the controller 11 thus collects the performance index calculated for a worker 50 regarding the basic operations in the proficiency database 70; hereby, the controller 11 creates a history of the performance indexes for the worker 50 regarding the basic operations.

### Step S105

Returning to FIG. 6, the history generated by the controller 11 acting as the index calculating unit 112 and repeatedly executing the above steps S102 through S104 represent past performance indexes; in step S105, the controller 11 uses the past performance indexes to predict a future performance index of the worker 50 regarding the basic operations.

The technique used to predict a future performance index from a history of past performance indexes may be selected as appropriate in accordance with the form of implementation. The controller 11 may use a linear regression model, a non-linear regression model, a multiple regression model, an autoregressive model (such as ARMA, ARIMA, SARIMA) to predict a future performance index from a past performance index. The future date and time for prediction is not particularly limited, and may be determined as appropriate in accordance with the form of implementation. For instance, the future date and time for prediction may be a predetermined value designated entered via input from the operator, or may be determined in accordance with the period at which the above steps S102 through S104 are repeatedly executed. A future prediction may also be for a date and time that the worker 50 is to carry out the work step assigned as determined in the later-described assignment mode. The prediction range may be in months, weeks, days, hours, or the like. The timing for predicting the performance indexes may be determined per cycle wherein the mean error between past predicted results and the measured results fit within a defined value. In this case, the prediction range may be a range wherein the mean error between past predicted results and the measured results fit within a defined value. The controller 11 continues to processing in step S106 once the prediction of future performance indexes is complete.

### Step S106

In step S106, the controller 11 acts as the history creation unit 116 and associates the worker 50, the work step 40, and the performance index for each of the basic operations forecast in step S105, and stores these in a predetermined storage area.

Similar to the above step S104, in this embodiment the controller 11 stores the performance indexes forecast for each of the basic operations in the proficiency database 70 in the storage unit 12. That is, the controller 11 creates a record in the proficiency database 70. The controller 11 stores information identifying the worker 50, information identifying the work step 40, information representing the date and time, the performance indexes calculated in step S103 for each of the basic operations in fields of the record created, and information indicating how the performance indexes were derived (in this step S106, "Predicted"). The controller 11 thus associates and stores the worker 50, the work step 40, and a performance index forecast for each basic operation in the proficiency database 70.

Herewith, the controller 11 completes a series of processes in the measurement mode according to this operational example. The controller 11 may execute the above-mentioned series of processes in steps S101 through steps S106 or S101 through S104 according to an instruction entered via input from an operator or the like to measure the performance index for the worker 50. The timing for executing the measurement mode in this operational example may be selected as appropriate in accordance with the form of implementation. The controller 11 may also repeat the series of steps S101 through S106 when a worker 50 performs the work step 40 to thereby measure the performance index of the worker 50 regarding the basic operations. The controller 11 may execute the processes in the above steps S101 through S106 for each of the workers on the roster at a production site to thereby measure the performance indexes for the workers regarding the basic operations in each of the work steps.

The processing sequence in the measurement mode according to the above operational example may be modified where possible. For example, the controller 11 may omit the processing in step S101 and execute the processing in steps S102 through S106. The controller 11 may also omit steps S105 and S106.

### Registration Mode

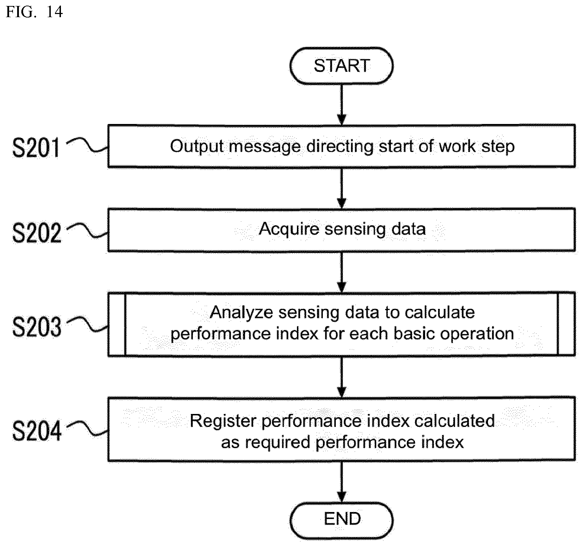

Next, an example of a processing sequence during the registration mode of a performance measurement device 1 according to the embodiment is described using FIG. 14; FIG. 14 is a flowchart illustrating an example of a processing sequence during the registration mode of a performance measurement device 1 according to the embodiment.

### Step S201 through S203

Except for where the worker 50 is a skilled operator, steps S201 through S203 are identical to the above steps S101 through S103. That is, in step S201, the controller 11 outputs a message directing the skilled operator to execute a target work step. In step S202, the controller 11 operates as the data acquisition unit 111 and uses one or a plurality of sensors to measure an activity (behavior) relating to at least one of sensory activity and physical activity of the skilled operator while the skilled operator executes the target work step whereby the controller 11 acquires the sensing data 121. A skilled operator is capable of suitably accomplishing at least the target work step among the plurality of work steps performed at the production site. Note that here, the target work step is an example of the "third work step." The target work step may be the same as the above work step 40 or may be different from the work step 40. In step S203, the controller 11 analyzes the sensing data 121 acquired from the skilled operator to thereby calculate the performance index of the skilled operator for each of the basic operations. Once the performance index is computed for the skilled operator, the controller 11 continues to performing the next step S204.

The controller 11 identifies the execution time, the time overlap, the number of executions, and the execution order for each of the basic operations during the process of deriving the performance index for each of the basic operations from the sensing data 121 of a skilled operator in step S203. The controller 11 may also establish the correct order and the suitable time (i.e., the exemplary behavior) for each of the above basic operations on the basis of the execution time, the time overlap, the number of executions, and the execution order for each of the basic operations during the process of the skilled operator accomplishing the work step. In this case, the controller 11 may terminate the processing sequence in the registration mode according to this operational example without calculating the performance index for the skilled operator.

If the correct order and a suitable time is already established for each of the basic operations, the controller 11 may revise or update the correct order and the suitable time for each of the basic operations on the basis of the execution time, the time overlap, the number of executions, and the execution order for each of the basic operations performed by the skilled operator. In this case, the controller 11 may also evaluate the correctness, stability, speed, and rhythm of execution of each of the basic operations performed by the skilled operator on the basis of the correct order and the suitable time already established for each of the basic operations. The controller 11 may continue to calculate the performance index for the skilled operator with respect to each of the basic operations on the basis of the results of this evaluation. The controller 11 may also revise or update the standard value for the performance index for each of the basic operations from the performance index calculated for the skilled operator with respect to each of the basic operations.

### Step S204

In step S204, the controller 11 operates as the registration unit 117 and registers the performance index computed for the skilled operator for each of the basic operations in step S203 as the required performance index for each of the basic operations.

As an example of the registration process, the controller 11 may store the performance index calculated for the skilled operator for each of the basic operations in step S203 as the required performance index for each of the basic operations in a usable form in a predetermined storage area. The predetermined storage area may be RAM, the storage unit 12, an external storage device, storage media, or a combination of these. The controller 11 may store the performance index computed for the skilled operator with respect to each of the basic operations as the required performance index in a usable format in the proficiency database 70. However, the particulars of the registration process are not limited to this example and may be determined as appropriate in accordance with the form of implementation. In the embodiment the controller 11 stores the performance index computed for skilled operator with respect to the basic operations in a work database 75 (later described).

The required performance indexes for the basic operations may be calculated from a plurality of skilled operators. For instance, the required performance index for the basic operations may be the mean of the performance indexes calculated for a plurality of skilled operators regarding the basic operations. Any of the plurality of skilled operators may be selected and the required performance index calculated for a basic operation from the performance index of the skilled operator who is selected.

Herewith, the controller 11 completes a series of processes in the registration mode according to this operational example. The controller 11 may execute the above-mentioned series of processes in steps S201 through S204 according to an instruction entered via input from an operator or the like to perform measurement during the registration mode. The controller 11 may execute the above-mentioned series of processes in steps S201 through S204 according to an instruction indicating the worker 50 is a skilled operator. The timing for executing the registration mode in this operational example may be selected as appropriate in accordance with the form of implementation.

The processing sequence for the registration mode according to the above operational example may be modified where possible. For example, the controller 11 may omit the processing in step S201 and execute the processing in steps S202 through S204.

### Assignment Mode

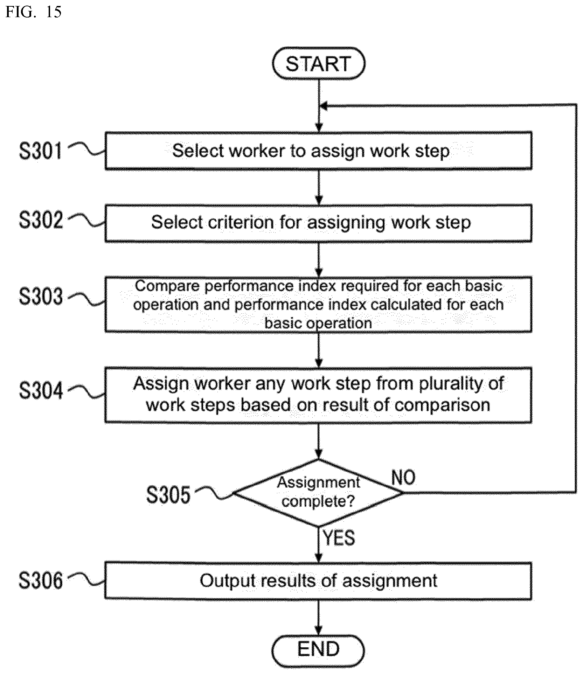

Next, an example of a processing sequence during the assignment mode of a performance measurement device 1 according to the embodiment is described using FIG. 15; FIG. 15 is a flowchart illustrating an example of a processing sequence during the assignment mode of a performance measurement device 1 according to the embodiment.

### Step S301

The controller selects the worker who is the target for assignment of a work step (e.g., the worker 50).

The method of selecting a target worker for assignment is not particularly limited and may be selected as appropriate in accordance with the form of implementation. For instance, the target worker may be selected via input from the operator. The controller 11 may also select the target worker from a list of individual workers on a roster at the production site. This list may be stored on the storage unit 12, or may be stored on an external storage device. The list may be acquired from the proficiency database 70 which stores the performance indexes for each of the workers. The controller 11 accesses the list as is appropriate and selects a target worker from the list obtained. The order in which a worker is selected by be determined as appropriate. For instance, the controller 11 may select a target worker in order from the top of the list, or may select a target worker randomly from the list. On selecting the target worker, the controller 11 continues to the processing in the next step S302.

### Step S302 through S303

In step S302 the controller 11 selects the criterion by which to assign a work step to the target worker. As the comparator unit 113 in step S303, the controller 11 compares the required performance index for the basic operations in the work steps and the performance index calculated for the target worker (i.e., the worker 50) in each of the basic operations. As the work assignment unit 114 in step S304, the controller 11 assigns the worker any work step among the plurality of work steps performed at a production site on the basis of the results of the comparison where the performance index calculated for the worker in each of the basic operations satisfies a criterion selected in step S302 in relation to the required performance index for each of the basic operations. The criterion selected in step S302 is one example of a "predetermined criterion".

### (1) Work Database

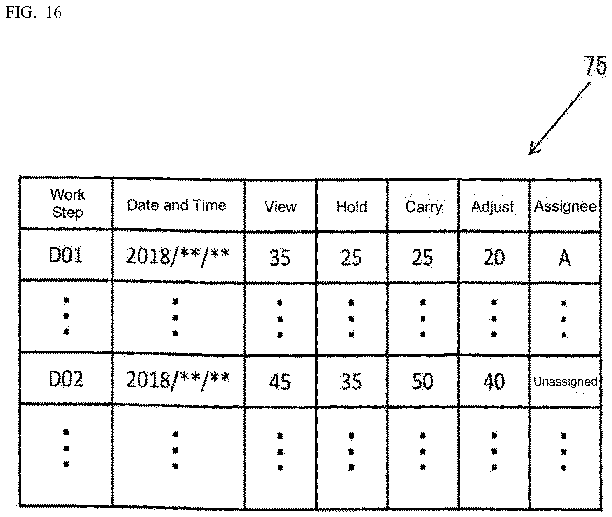

First, the work database 75 is described with further reference to FIG. 16; the work database 75 stores the required performance index for the work steps performed at the production site. FIG. 16 schematically illustrates an example of a work database 75 according to the embodiment. In the example in FIG. 16, the work database 75 is expressed in table form where each record (row data) includes the fields: Work Step, Date and Time, View, Hold, Carry, Adjust, and Assignee.

The work step field stores information for identifying the target work step. The date-and-time field stores information representing the date and time the target worker is required to accomplish the target work step. The assignee field stores information identifying the worker to which the target work step is assigned. Note that in the example in FIG. 16, an assignee field that includes "Unassigned" indicates that no worker is currently assigned to the work step and date and time in the record.

The View, Hold, Carry, and Adjust fields store the required performance index for each of these basic operations. In the above mentioned step S204 the controller 11 stores the performance indexes calculated for the skilled operator performing each of the basic operations in each of the fields: View, Hold, Carry, and Adjust contained in a record corresponding to the work step (third work step) performed by the skilled operator. However, the method of establishing a required performance index for each of the basic operations in a work step is not limited to this kind of example. The required performance indexes for each of the basic operations may be designated by input from an operator.

Hereby each of the records in the work database 75 can represent the assignment of a specific worker to a specific work step and specific date and time (a single assignment). However, the data format and the fields in the work database 75 are not limited to this type of example and may be selected is appropriate in accordance with the form of implementation.

The controller 11 may extract records where the assignee field contains information indicating that no worker is yet assigned (i.e., "Unassigned", FIG. 16) to acquire candidate work steps to which a target worker may be assigned. The controller 11 may also reference the View, Hold, Carry, and Adjust fields in each of the records to acquire the required performance indexes for each of the basic operations in each of the work steps.

### (2) The Performance Index for a Worker

The performance index of the worker, which is compared to the required performance index, is described next. In this embodiment, the processing in the above step S103 calculates the performance index of a worker for each of the basic operations included in the work step 40. The processing in the above step S105 predicts the future performance index of a worker in each of the basic operations. The controller 11 compares either of these performance indexes with the required performance index.

That is, in step S303, the controller 11 may compare the performance index calculated in step S103 for the worker for each of the basic operations with the required performance index for each of the basic operations in each of the work steps immediately before executing assignment processing. Alternatively, the controller 11 may compare the future performance index predicted for the worker in each of the basic operations with the required performance index for each of the basic operations in each of the work steps when a future performance index is acquired for a date and time the worker is to accomplish the work step that is assigned.

### (3) The Assignment Criterion

Criteria under which a target worker may be assigned a work step is described. The required performance index for each of the basic operations in each of the work steps is used as a referenced for determining whether or not a worker has the ability to complete a target work step at a standard quality within an established time. In other words, if the worker has a performance index that is higher than the required performance index for all the basic operations, then the worker is evaluated as having the ability to complete the target work step at a standard quality within an established time. Therefore, in terms of maintaining the productivity at a production site, the worker with a performance index that is higher than the required performance index is preferably assigned all the work steps.

On the other hand, all the workers on the roster at a particular production site do not necessarily have a high performance index. As illustrated in the above FIG. 10, a beginner learns the work step in the process of repeatedly performing the work step, to thereby learn the skills to complete said work step at a standard quality in a standard time. In terms of encouraging this kind of inexperienced worker to master the work step, it may be important in some cases to assign the target work step to a worker with a performance index that is lower than the required performance index. However, too great a difference between the performance index of the worker and the required performance index may result in the worker's skill being too far below the skill required to suitably complete the work step, and it is very likely it is not possible to encourage the worker to master this work step. In terms of encouraging this kind of inexperienced worker to master the work step, it may be preferable to assign the target work step to a worker with a performance index that is slightly lower than the required performance index. Based on this perspective, the controller 11 in this embodiment determines the work step to assign to a worker on the basis of any of the following two criteria.

### (3-1) First Assignment Criterion

The first assignment criterion is described first. The first assignment criterion is that the difference between the performance index of the worker and a required performance index for each of the basic operations is at or below a predetermined value (threshold). That is, the controller 11 determines whether or not the difference between a required performance index for each of the basic operations in any work step among the plurality of work steps and the performance index calculated for a worker in each of the basic operations is at or below a predetermined value. On determining that the difference between the performance index required for each of the basic operations in any work step and the performance index calculated for the worker in each of the basic operations is at or below the predetermined value, the controller 11 assigns said work step to the worker.

The first assignment criterion allows a target work step to be assigned to a worker having a performance index that is close to the required performance index. Therefore, this prevents a worker having exceptionally high ability being assigned an extremely easy work step, and prevents a worker with an exceptionally low ability from being assigned an extremely difficult work step. Hereby, it is possible to maintain the productivity of a production line at a production site.

Note that the difference between the performance index of the worker and the required performance index may be a representative value of the difference for any basic operation, or may be obtained from the sum (absolute value) of the differences for all the basic operations. The aforementioned difference may also be obtained as the distance between the performance index of the worker and the required performance index. Additionally, when attempting to optimize calculations, the controller 11 may assign workers to work steps so that the sum of the differences for each of the workers is a minimum for optimization purposes.

### (3-2) Second Assignment Criterion

The second assignment criterion is described next. The second assignment criterion is that the performance index of the worker is lower than the required performance index for at least any of the basic operations, and the difference between the performance index of the worker and the required performance index for the basic operations is a minimum. That is, the controller 11 assigns the worker a work step from a plurality of work steps where the performance index calculated for the worker in at least any basic operation among the plurality of basic operations is lower than the performance index that is required for said basic operation, and the difference between the performance index calculated for the worker in each of the basic operations and the required performance index for each of the basic operations is a minimum.

The second assignment criterion allows a target work step to be assigned to a worker who does not satisfy the standard for suitably accomplishing any of the basic operations in the target work step but whose performance index approaches the required performance index. In other words, a worker having a performance index that is slightly lower than the required performance index may be assigned to the target work step and thereby an inexperienced worker may be encouraged to suitably master a work step (more specifically, any of the above-mentioned basic operations).

Similar to the above first assignment criterion, the difference between the performance index of a worker in each of the basic operations and the required performance index for each of the basic operations may be a representative value of the difference for any basic operation, or may be obtained from the sum (absolute value) of the differences for all the basic operations. The aforementioned difference may also be obtained as the distance between the performance index of the worker and the required performance index.

### (3-3) Additional Assignment Criteria

The criterion for assigning a work step to a target worker is not limited to the above mentioned two criteria and may be selected is appropriate in accordance with the form of implementation. For example, the controller 11 may subtract the performance index required for each of the basic operations for any work step among the plurality of work steps from the performance index calculated for the worker in each of the basic operations to thereby obtain a difference; and the controller 11 may determine whether or not the difference is at or above a predetermined value. The controller 11 may also assign any work step to a worker when the controller 11 determines that said difference is at or above the predetermined value. The predetermined value may be zero or greater, or maybe a negative value. Additionally, the controller 11 may assign a work step to a worker if the difference between the sum of the performance indexes calculated for a worker in each of the basic operations and the sum of the required performance indexes is a minimum. The period for which the worker is engaged in the assigned work step may be in months, weeks, days, hours, or the like.

Herewith the controller 11 executes the processes in step S302 through step S304 to assign a target worker to any work step among a plurality of work steps performed at a production site. Once any work step from the plurality of work steps is assigned to the target worker, the controller 11 continues to the processing in the next step S305.

The controller 11 may selected any of the above two assignment criteria in the above step S302. This selection may be made automatically or made manually via input from an operator. The controller may also store information for identifying the worker assigned the target work step in the assignee field of the relevant record in the above step S304. Hereby, the work step assigned to the target worker is saved in the work database 75.

### Step S305

In step S305 the controller determines whether or not the process of assigning each work step to a worker is complete. On determining that assignment is not complete, the controller repeats processing from step S301. Whereas, on determining that assignment is complete, the controller continues to step S306.

The process of determining whether or not assignments are complete can be made as appropriate. For example, the controller 11 may determine that the each of the work steps are assigned to a worker when the assignment of all the work steps performed at a production site to workers available for a predetermined period is complete. In contrast, when it is not the case, the controller 11 may determine that the assignment of the workers to each of the work steps is incomplete. The controller 11 references each of the records in the work database 75 to determine whether or not the work steps performed at a production site are assigned to a worker. The predetermined period may be designated as appropriate, for instance, via input from an operator.

### Step S306

In step S306 the controller 11 functions as the output unit 115 and outputs the results of the assignments. The destination and method of output may be performed as appropriate in accordance with the form of implementation. For example, the controller may generate a list indicating the assignments of workers to work steps and the period during which the assigned worker is to perform the work step; the controller 11 may output this list to the output device 15. The controller may also transmit this list to a user terminal carried by the worker 50 or a supervisor.

Herewith, the controller 11 completes a series of processes in the assignment mode according to this operational example. The controller may execute the series of processes in the above step S301 through S306 in accordance with an operator input directing assignments of workers to work steps via the assignment mode. The controller 11 may also execute the series of processes in the above steps S301 through S306 regularly or irregularly. The timing for executing the assignment mode in this operational example may be selected as appropriate in accordance with the form of implementation. The processing sequence in the assignment mode according to the above operational example may be modified where possible. For example, step S302 may be omitted if there are permanent assignment criteria.

### Features

As above described, a performance measurement device 1 according to the embodiment performs the above steps S101 through S103 to thereby compute the performance index of a worker 50 from the sensing data 121 for each of the basic operations included in a work step 40. Given that each of the performance indexes correspond to the degree of the ability to accomplish each of the basic operations, the performance indexes computed can objectively and quantitatively indicate the ability of a worker 50 with respect to each of the basic operations.

In this embodiment, the performance measurement device 1 compares the required performance index for each of the basic operations in each of the work steps with the performance index calculated for the worker in each of the basic operations; the performance measurement device 1 further assigns any work step among the plurality of work steps performed at a production site to a worker on the basis of the comparison results. Thus, the performance measurement device 1 according to the embodiment uses the results of evaluating the performance index to assign the aforementioned worker a work step for which the worker is suited.

Therefore, the performance measurement device 1 according to the embodiment can objectively and quantitatively measure the ability of a worker 50 to perform a work step 40. The performance measurement device 1 according to the embodiment may be configured to treat a worker at a production site as the worker 50, measuring the performance index for the worker and assigning the worker to a work step on the basis of the performance index measured. Thus, the present embodiment can more appropriately assign workers to work steps. Therefore, it is possible to systematically improve or maintain the productivity and efficiency of the production line at the production site. Moreover, even if a worker has no experience with all the work steps at a production site, the embodiment allows for a work step to be assigned to a worker suited thereto on the basis of knowledge obtained through the worker executing any work step (i.e., through the performance index calculated for the basic operations). Therefore, it is possible to reduce the cost associated with optimizing assignments of a worker to a work step.

### 4. Modification Examples

While an embodiment of the present invention is described above in detail, all points in the previous description are merely examples of the present invention. It goes without saying that various modifications and variations are possible without departing from the scope of the invention. For instance, the following modification is possible. Note that constituent elements that are identical to the constituent elements in the above described embodiment are given the same reference numerals and where appropriate, a description of features that are identical to the above embodiment are omitted. The following modifications may be combined as appropriate.

### 4.1

In the above embodiments an EEG 30 and a load cell 31 are used for measuring the sensory activity on the physical activity of the worker 50. However, the number of sensors used for measuring at least one of the sensory activity on the physical activity is not limited to two; there may be one sensor or three or more sensors.

The sensor is also not limited to an EEG 30 and a load cell 31. As long as the sensor is capable of measuring a physiological parameter relating to at least one of the sensory activity and the physical activity of the target operator, the type of sensor is not particularly limited and may be selected as appropriate in accordance with the form of implementation. The behavior of the sensory systems may be expressed for instance through brain waves, cerebral blood flow, pupil diameter, gaze direction, facial expression, voice, electrocardiogram, blood pressure, electromyograph, a galvanic skin reflex, or the like. Therefore, the one or plurality of sensors for measuring sensory activity may be one or a combination of, for example: an electroencephalographic meter; a magnetoencephalographic meter; a magnetic resonance imaging device configured to use functional magnetic resonance imaging to take images of the blood flow in relation to brain activity; a brain activity measurement device configured to use functional near infrared spectroscopy to measure cerebral blood flow; a gaze sensor configured to measure pupil diameter and gaze direction; an electrooculographic sensor; a microphone; an electrocardiographic meter, a blood pressure meter; an electromyographic sensor; a galvanic skin reflex meter; and a camera. In contrast, the behaviors and physical activities may be expressed with the musculoskeletal system, such as the finger, hand, leg, neck, waist, joints, or muscles. Therefore, the one or plurality of sensors for measuring physical activity may be for instance a camera, motion capture device, load cell or a combination of these. Therefore, the one or plurality of sensors may be made up one or a combination of a camera, a microphone, an electroencephalography meter, a magnetoencephalographic meter, a magnetic resonance imaging device, an electrocardiography meter, a blood pressure meter, a galvanic skin reflex meter, an electromyographic sensor, a load cell, a motion capture device, a brain activity measurement device, a gaze sensor, and an electrooculographic sensor.

The sensing data 121 according to the above embodiment is made up of a first measurement data containing the amount of brain activity measured by the EEG 30; and a second measurement data containing the load measured by the load cell 31. However, the composition of the sensing data 121 is not limited to this kind of example; the sensing data may be determined as appropriate in accordance with the type of sensor used to measure activities related to at least one of the sensory activity and physical activity of the target operator.

In the above-mentioned embodiment, the performance measurement device 1 was also connected to an EEG 30 and a load cell 31 via the external interface 13; however, the performance measurement device 1 may acquire sensing data 121 directly from the EEG 30 and the load cell 31. However, the method of acquiring the sensing data 121 is not particularly limited to this example and may be selected as appropriate in accordance with the form of implementation. For example, the one or plurality of sensors may be connected to an information processing device other than the performance measurement device 1. In this case, the performance measurement device 1 (the controller 11) may acquire the sensing data 121 indirectly from the other information processing device via the network or a recording medium, or the like.

### 4.2

In the above embodiment, the proficiency database 70 and the work database 75 are stored in the storage unit 12. However, the location for storing the proficiency database 70 and the work database 75 are not limited to the above example; the storage location may be selected as appropriate in accordance with the form of implementation. At least one of the proficiency database 70 and the work database 75 may be stored on an external storage device such as a NAS, or the like. In this case, the performance measurement device 1 may access the at least one of the proficiency database 70 and the work database 75 that is stored on an external storage device via a network or the like.

### 4.3

The basic operations of "View", "Hold", "Carry", "Adjust" in the above embodiment are defined to include the human cognitive process for at least one cycle in the above embodiment. However, the definition of each of the basic operations is not limited to this example and may be determined as appropriate in accordance with the form of implementation as long as it is possible to identify the basic operations within a work step. Additionally, the type of basic operations is not limited to the above four types of basic operations; any of the basic operations may be omitted, any of the basic operations may be replaced, and different types of basic operations may be added as appropriate in accordance with the form of implementation.

### 4.4

The performance measurement device 1 according to the above embodiment uses for metrics: correctness, stability, speed, and rhythm for evaluating the precision of executing each of the basic operations. However, the processing in step S103 is not limited to this example and may be determined as appropriate in accordance with the form of implementation. For example, the controller 11 may evaluate any one of the correctness, stability, speed, and rhythm of executing each of the basic operations in the above step S1304; and in the above step S1305, the controller may calculate performance index for each of the basic operations in accordance with the results of said evaluation.

### Reference Numerals

- 1: Performance measurement device
- 11: Controller
- 12: Storage unit
- 13: External interface
- 14: Input device
- 15: Output device
- 16: Drive
- 111: Data acquisition unit
- 112: Index calculating unit
- 113: Comparator
- 114: Work assignment unit
- 115: Output unit
- 116: History creation unit
- 117: Registration unit
- 80: Performance measurement program
- 121: Sensing data
- 70: Proficiency database
- 75: Work database
- 90: Recording medium
- 30: EEG
- 31: Load cell
- 40: Work step
- 50: Worker

## Claims

1. A performance measurement device (1) comprising: a data acquisition unit (111) configured to acquire sensing data (121) obtained from one or a plurality of sensors (30, 31) configured to measure an activity relating to at least one of sensory activity and physical activity of a worker (50) while the worker (50) executes a first work step among a plurality of work steps performed at a production site, each work step (40) including a plurality of basic operations;
an index calculating unit (112) configured to analyze the sensing data (121) acquired and thereby use the sensing data (121) to calculate a performance index for the worker (50) in each of the basic operations, the performance index indicating a degree of performance of the basic operations achieved through said activity;
a comparator unit (113) configured to compare the performance index required for each of the basic operations in a work step (40) to suitably accomplish the work step (40), and the performance index calculated for the worker (50) in each of the basic operations;
a work assignment unit (114) configured to assign the worker (50) a second work step (40) from the plurality of work steps on the basis of the results of the comparison, where the performance index calculated for the worker (50) in each of the basic operations satisfies a predetermined criterion in relation to the performance index required for each of the basic operations; and
an output unit (115) configured to output the result of the assignment.

2. The performance measurement device (1) according to claim 1, wherein the data acquisition unit (111) repeatedly acquires sensing data (121) for the worker (50);
the index calculating unit (112) calculates the performance index for the worker (50) in each of the basic operations from the sensing data (121) each time sensing data (121) is acquired; and
the performance measurement device (1) further including: a history creation unit (116) configured to accumulate the performance indexes calculated for a worker (50) in each of the basic operations to thereby create a history of performance indexes for the worker (50) in each of the basic operations;
the index calculating unit (112) predicts a future performance index for the worker (50) in each of the basic operations from past performance indexes represented by the history created; and
the comparator unit (113) compares the performance index required for each of the basic operations in each of the work steps and the future performance index predicted for the worker (50) in each of the basic operations .

3. The performance measurement device (1) according to claim 1 or 2, wherein: the data acquisition unit (111) uses one or a plurality of sensors to measure the activity of a skilled operator able to suitably accomplish a third work step among the plurality of work steps while the skilled operator executes the third work step to thereby acquire sensing data (121);
the index calculating unit (112) analyzes the sensing data (121) acquired from the skilled operator to thereby calculate a performance index for the skilled operator in each of the basic operations; and
the performance measurement device (1) further comprising: a registration unit (117) configured to register the performance index calculated for the skilled operator in each of the basic operations in the third work step as the performance index required for each of the basic operations.

4. The performance measurement device (1) according to any one of claims 1 through 3, wherein: each of the basic operations is defined to include a human cognitive process for at least one cycle; and
the data acquisition unit (111) acquires sensing data (121) obtained by using a plurality of sensors to measure the sensory activity and the physical activity of the worker (50).

5. The performance measurement device (1) according to any one of claims 1 through 4, wherein: analyzing the sensing data (121) includes evaluating at least any one of the correctness, speed, stability, and rhythm of executing the basic operations; and
the index calculating unit (112) calculates the performance index for the worker (50) in each of the basic operations in accordance with the result of the evaluation.

6. The performance measurement device (1) according to claim 5, wherein: analyzing the sensing data (121) includes:
converting the sensing data (121) into time series feature data (S1301);
analyzing the time series feature data to thereby identify the execution time, time overlap, number of executions, and execution order of the basic operations (S1302, S1303); and
evaluating the correctness, speed, stability, and rhythm of execution for each of the basic operations on the basis of the execution time, time overlap, number of executions, and execution order for the basic operations (S1304); and
the index calculating unit (112) calculates the performance index for the worker (50) in each of the basic operations in accordance with the result of the evaluation (S1305).

7. The performance measurement device (1) according to any one of claims 1 through 6, wherein: the work assignment unit (114)
determines whether or not the difference between the performance index calculated for a worker (50) in each of the basic operations is at or below a predetermined value compared to the performance index required for each of the basic operations in any work step (40) among the plurality of work steps; and
assigns the worker (50) any work step as a second work step on determining that the difference between a performance index required for each of the basic operations in said work step (40) is at or below the performance index calculated for the worker (50) in each of the basic operations.

8. The performance measurement device (1) according to any one of claims 1 through 7, wherein: the work assignment unit (114) assigns the worker (50) a work step (40) from among the plurality of work steps as said second work step where the performance index calculated for the worker (50) in at least any basic operation among the plurality of basic operations is lower than the performance index required for said basic operation, and the difference between the performance index calculated for the worker in each of the basic operations and the performance index required for each of the basic operations is a minimum.

9. The performance measurement device (1) according to any one of claims 1 through 8, wherein: the one or plurality of sensors is made up of one or a combination of a camera, a microphone, an electroencephalographic meter (30), a magnetoencephalographic meter, a magnetic resonance imaging device, an electrocardiographic meter, a blood pressure meter, a galvanic skin reflex meter, an electromyographic sensor, a load cell (31), motion capture, a brain activity measurement device, a gaze sensor, and an electrooculographic sensor.

10. A performance measurement method causing a computer to execute steps comprising:
acquiring sensing data (121) obtained from one or a plurality of sensors configured to measure an activity relating to at least one of sensory activity and physical activity of a worker (50) while the worker (50) executes a first work step among a plurality of work steps performed at a production site, each work step including a plurality of basic operations (S102; S202);
analyzing the sensing data (121) acquired and thereby using the sensing data (121) to calculate a performance index for the worker (50) in each of the basic operations, the performance index indicating a degree of performance of the basic operations achieved through said activity (S103; S203);
comparing the performance index required for each of the basic operations in a work step to suitably accomplish the work step (40), and the performance index calculated for the worker (50) in each of the basic operations (S303);
assigning the worker (50) a second work step from the plurality of work steps on the basis of the results of the comparison, where the performance index calculated for the worker (50) in each of the basic operations satisfies a predetermined criterion in relation to the performance index required for each of said basic operations (S304); and
output of the result of the assignment (S306).

11. A performance measurement program (80) causing a computer to execute steps comprising:
acquiring sensing data (121) obtained from one or a plurality of sensors configured to measure an activity relating to at least one of sensory activity and physical activity of a worker (50) while the worker (50) executes a first work step among a plurality of work steps performed at a production site, each work step including a plurality of basic operations (S102; S202);
analyzing the sensing data (121) acquired and thereby using the sensing data (121) to calculate a performance index for the worker in each of the basic operations, the performance index indicating a degree of performance of the basic operations achieved through said activity (S103; S203);
comparing the performance index required for each of the basic operations in a work step (40) to suitably accomplish the work step (40), and the performance index calculated for the worker (50) in each of the basic operations (S303);
assigning the worker (50) a second work step from the plurality of work steps on the basis of the results of the comparison, where the performance index calculated for the worker in each of the basic operations satisfies a predetermined criterion in relation to the performance index required for each of said basic operations (S304); and
output of the result of the assignment (S306).
